# EUROPEAN PATENT SPECIFICATION

(11) **EP 1 789 435 B1**
(45) Date of publication and mention of the grant of the patent: **24.02.2010**
(21) Application number: 06798754.5
(22) Date of filing: 12.09.2006
(51) Int. Cl.: C12N 5/07, C07K 14/745

(54) **A METHOD FOR PRODUCTION OF MATURE NATURAL KILLER CELL**
VERFAHREN ZUR HERSTELLUNG EINER VOLL ENTWICKELTEN NATÜRLICHEN KILLERZELLE
METHODE DE PRODUCTION DE CELLULE TUEUSE NATURELLE MATURE

(30) Priority: 12.09.2005 KR 20050084896; 12.09.2005 KR 20050084897; 12.09.2005 KR 20050084898; 26.07.2006 KR 20060070390
(43) Date of publication of application: 30.05.2007
(73) Proprietor: Industry Foundation of Chonnam National University, Buk-gu Gwangju 500-757 (KR); Good Cell Life, Inc., Guro-gu, Seoul 152-740 (KR)
(72) Inventor: KANG, Hyung Sik, Gwangju 500-793 (KR); KIM, Sang Hyun, Seoul 133-112 (KR); KIM, Eun Mi, Gwangju 500-060 (KR); LEE, Eun Hee, Gwangju 502-765 (KR); YUN, Hee Jung, Jeollanam-do 525-814 (KR); KO, Chang Bo, Chungcheongnam-do 314-140 (KR); HEO, Young Jin, Seoul 158-070 (KR); SHIM, Woo Young, Incheon 403-100 (KR); HEO, Seong Beom, Seoul 121-240 (KR); YOON, Ji Yeon, Seoul 152-050 (KR)
(74) Representative: Gillet, Raphaëlle
(86) International application number: PCT/KR2006/003627
(87) International publication number: WO 2007/032634

(56) References cited:
- KIM EUN-MI ET AL: "Role of Axl receptor tyrosine kinase in the development and effector function of natural killer cells" JOURNAL OF IMMUNOLOGY, vol. 176, no. Suppl. S, April 2006 (2006-04), page S269, XP008089540 & ANNUAL MEETING OF THE AMERICAN-ASSOCIATION-OF-IMMUNOLOGISTS; BOSTON, MA, USA; MAY 12 -16, 2006 ISSN: 0022-1767
- WALZER THIERRY ET AL: "NK cell development: gas matters." NATURE IMMUNOLOGY JUL 2006, vol. 7, no. 7, July 2006 (2006-07), pages 702-704, XP002472979 ISSN: 1529-2908
- CARAUX ANOUK ET AL: "Natural killer cell differentiation driven by Tyro3 receptor tyrosine kinases." NATURE IMMUNOLOGY JUL 2006, vol. 7, no. 7, July 2006 (2006-07), pages 747-754, XP002472980 ISSN: 1529-2908
- MCCLOSKEY P. ET AL.: 'GAS6 Mediates Adhesion of Cells Expressing the Receptor Tyrosine Kinase Axl' THE JOURNAL OF BIOLOGICAL CHEMISTRY vol. 272, no. 37, 1997, pages 23285 - 23291, XP003010241
- WILIAMS N.S. ET AL.: 'Differentiation of NK1.1+, Ly49+ NK Cells from flt3+ Multipotent Marrow Progenitor Cells' J. IMMUNOL. vol. 163, no. 5, 01 September 1999, pages 2648 - 2656, XP003010242
- COLUCCI F. ET AL.: 'The receptor tyrosine kinase c-kit provides a critical signal for survival, expansion, and maturation of mouse natural killer cells' BLOOD vol. 95, no. 3, 01 February 2000, pages 984 - 991, XP003010243
- CARAYOL G. ET AL.: 'NK CELLS DIFFERENTIATED FROM BONE MARROW, CORD BLOOD AND PERIPHERAL BLOOD STEM CELLS EXHIBIT SIMILAR PHENOTYPE AND FUNCTIONS' EUR. J. IMMUNOL. vol. 28, no. 6, June 1998, pages 1991 - 2002, XP002406970

## Description

### Technical field

The present invention relates to a ligand of Axl receptor tyrosine kinase used to induce the differentiation from precursor natural killer cell to mature natural killer cell. In addition, it relates to a process for producing mature natural killer cell comprising treating hematopoietic stem cell with interleukin-7, stem cell factor and FIt3L to differentiate into precursor natural killer cell, and treating the resulting precursor natural killer cell with ligand of Axl receptor tyrosine kinase to produce mature natural killer cell.

### Background Art

The present invention relates to a ligand of Axl receptor tyrosine kinase (hereinafter to be referred to as Axl protein) used to induce the differentiation precursor natural killer (pNK) cell to mature natural killer cell. More particularly, it relates to a process for producing mature natural killer cell comprising treating hematopoietic stem cell with interleukin-7, SCF (stem cell factor) and F1t3L (FMS-like tyrosine kinase 3 ligand) to differentiate into precursor natural killer cell (pNK), and treating the resulting precursor natural killer cell with ligand of Axl protein to produce mature natural killer cell.

The modem-day approach to cancer management is a multidisciplinary one, consisting primarily of surgery, radiation therapy and chemotherapy, in varying combinations. However, all three of these treatments function within temporary effect and have problems to induce resistance, recurrence or adverse effects. Several advanced countries are actively developing immunotherapy in order to develop anticancer drugs and diagnostic reagents which can modulate immunological reaction, but enormous amount of research remains to be done and development is still in its infancy. Natural killer cells (NK cells) are lymphocytes of innate immunity that remove pathogenic, cancerous, and allogeneic cells. NK cells mediate adaptive immunity by secreting cytokines such as interferon-γ tumor necrosis factor-alpha, and IL-12 and have the ability to control specifically memorizing cancer. Previous cancer treatment utilizing NK cells involves enhancing immune response against cancer cells by activating NK cells with interleukin-2. However, this therapy is known to have several problems with adverse effects and differences in efficacy, tolerance and persistence among individuals. In order to increase NK cell's cancer destroying effect, it is the utmost importance to have a great amount of NK cells with high activity. Approximately more than ten billion (10¹⁰) NK cells are necessary for clinical treatment because NK cells have a possibility of application for treatment of cancer, incurable virus infection, etc. However, NK cells are very difficult to amplify. Various methods have been employed (e.g. Carayol G et al, Eur. J. Immunol. 1998.28:1991-2002) but only several tens of amplification was achieved by using IL-2 or IL-15, and that is a limit up to now. However, it is known that several hundreds of amplification is possible if cancer cells of other people are mixed with. According to the most recent method for amplifying NK cells, which was published in 2005, one thousand times of amplification is achieved when cancer cells of other people in which two genes are added are mixed with. However, it is still under study. For the amplification of at least several hundred times, it is a basic thing to do to culture cells by mixing cancer cells of other people. Still, there are problems of safety and ethical concerns for mixing cancer cells or using genes of other people.

### Disclosure of Invention

### Technical Problem

The object of the present invention is to provide mature NK cells in large quantity by inducing the differentiation from precursor NK cells to mature NK cells.

Another object of the present invention is to provide a substance which induces the differentiation from precursor NK cells to mature NK cells.

Yet another object of the present invention is to provide activated mature NK cells which are obtained from mature NK cells by treating with low dose of IL-2.

Yet another object of the present invention is to provide an immune cell therapeutic composition containing mature NK cells differentiated and activated in accordance with the present invention.

### Technical Solution

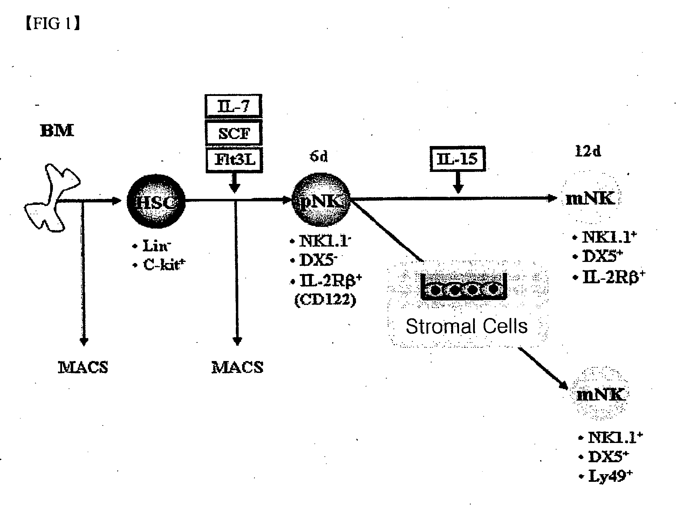

In order to achieve the technical subject described above, the present inventors investigated differentiation process of hematopoietic stem cells (HSCs) into mature natural killer (mNK) cells (Fig 1), different from the studies of prior art which mainly uses mNK cells. As a result, the inventors found Axl gene which promotes the differentiation. Axl (p140) is a tyrosine receptor phosphorylase belonging to a family of Sky and Eyk. In the family of Sky and Eyk, there are Rse (Sky, Brt, Tif, Dtk, Tyro3) and Mer(Eyk, Nyk, Tyro 12). It is known that Axl, Rse and Mer are expressed in most of tissues, but their function remains unknown. Gas6 is known as a ligand of Axl protein and is vitamin K-dependent potentiating factor and believed to be involved with migration, growth, and differentiation of Axl considering it specific structure. It has been reported that Axl protein is expressed in fibroblast, myeloidprogenitor, macrophage, neural tissues, follicle, and skeletal muscle but not in lymphocytes. Although Ax1 and Gas6 are known to regulate homeostasis of antigen presenting cells and growth of hematopoietic cells, the regulation of the development and function of NK cell by Axl and Gas6 are not known. The inventors of the present invention found that, Axl protein plays an essential role in differentiation of pNK cells into mNK cells, and based on such finding of the function of Axl protein as a differentiation regulator, isolated hematopoietic cells from mouse bone marrow and then established a system by which said hematopoietic cells are differentiated into mNK cells using Axl antibody and/or Gas protein. Further, by modifying said differentiation system and applying it to hematopoietic cells isolated from human peripheral blood, bone marrow or umbilical cord blood, mNK cells with enhanced activity are successfully produced in a large amount.

Following the success described above, in one aspect, the present invention provides a ligand for Axl protein which induces the differentiation of hematopoietic cells into mNK cells. As used herein, the term "ligand" means a polypeptide or a compound which binds Axl protein and thus induces an agonistic response of the receptor protein. Ligand of the present invention includes an antibody against Axl protein, Gas6 protein and protein S. The term "differentiation" means a phenomenon that a relatively simple system is divided into at least two partial systems which are qualitative different from the original system. In other words, the differentiation means a phenomenon in which a structure or function becomes specialized. In mNK cells, the term 'maturation' means that NK cells become to exert their intrinsic cellular function; for example, recognizing and directly killing cancer cells. Maturation of NK cells can be confirmed by using expressed substance as a marker. For a mouse or a human, it is well established. A mouse marker includes for example, NK1.1, CD122, LY49 Family (Ly49A, Ly49C, Ly49D, Ly49E, Ly49F, Ly49G, Ly49H, and Ly49I), NKG2A/C/E, and a human marker includes for example NKG2A, NKG2D, NKp30, NKp44, NKp46, CD56, and CD 161. Function of these markers is well known to a skilled person in the pertinent art.

In another aspect, the present invention provides a process of producing mNK cells, **characterized in that** (i) hematopoietic cells are treated with IL-7, SCF and Flt3L to differentiate into precursor NK cells, and (ii) the resulting precursor NK cells are treated with a ligand of Axl protein to differentiate into mNK cells, and therefore obtaining mNK cells.

In one embodiment, the present invention provides a process of producing human mNK cells **characterized in that** hematopoietic cells are isolated from human peripheral blood, bone marrow or umbilical cord blood.

In another embodiment, the present invention provides a process of producing human mNK cells **characterized in that** a ligand of Axl protein is selected from a group consisting of an antibody against Axl protein, human γ-carboxylated Gas 6 and mixtures thereof.

In another embodiment, the present invention provides a process of producing human mNK cells **characterized in that** precursor mNK cells are treated with the ligand in the presence of human stromal cell.

In another aspect, the present invention provides a process of producing activated mNK cells, **characterized in that** (i) treating hematopoietic cells with IL-7, SCF and Flt3L to differentiate them into precursor mNK cells, (ii) treating the resulting precursor mNK cells with the ligand of Axl protein to differentiate them into mNK cells, and therefore obtaining mNK cells, and (iii) activating the differentiated mNK cells with the treatment of IL-2. In one embodiment, the present invention provides a process **characterized in that** the differentiated mNK cells are treated with about 8 to 15ng/ml of IL-2.

In another aspect, the present invention provides an immune cell therapy composition **characterized in that** it comprises the activated mNK cells prepared by the process of the present invention. In the text of the present invention, it is understood that the immune cell therapy composition can be used as various anticancer agent or immunostimulant, etc. The term "activation" in the activated mNK cells means that after being stimulated by IL-2 mNK cells show a substantial cytotoxic effect.

In another aspect, the present invention provides an autoimmune cell therapy **characterized in that,** hematopoietic cells are isolated from blood and/or bone marrow of a patient to be treated, the resulting hematopoietic cells are treated with IL-7, SCF and Flt3L to differentiate them into precursor mNK cells, the resulting precursor mNK cells are treated with the ligand for Axl protein to differentiate them into mNK cells, the resulting mNK cells are activated with the treatment of IL-2, and the resulting activated mNK cells are introduced back to the patient to be treated. As used herein, "autoimmune cell therapy" means that the patient's own immune cells isolated from the body of the patient or immune cells obtained in vitro via differentiation using the patient's own hematopoietic cells, which can selectively destroy cancer cells, are amplified or strengthened via in vitro culture, and then the cells are re-introduced into the patient's own body in order to treat cancer.

In another aspect, the present invention provides an alloimmune cell therapy **characterized in that,** hematopoietic cells are isolated from human alloumbilical cord blood, donated blood and/or bone marrow, the resulting hematopoietic cells are treated with IL-7, SCF and Flt3L to differentiate them into precursor mNK cells, the resulting precursor mNK cells are treated with the ligand for Ax1 protein in the presence of a human stromal cell to differentiate into mNK cells, the resulting mNK cells are activated with the treatment of IL-2, and the resulting activated mNK cells are introduced to the patient to be treated. As used herein, "alloimmune cell therapy" means that fetal umbilical cord blood is differentiated into immune cells by using adult stem cells in vitro, thus the immune cells which can selectively destroy cancer cells are amplified or strengthened and are introduced to a patient's body to treat cancer.

Natural killer (NK) cells are white blood lymphocytes involving in the innate immune system that have diverse biological functions including recognition and destruction of certain microbial infections and neoplasms (Moretta, A., Bottino, C., Mingari, M.C., Biassoni, R. and Moretta, L., Nat. Immunol., 3, 6, 2002). Their morphological characteristic showed a large granular lymphocyte (LGL)-like morphology based on the presence of densely staining azurophilic granules in their cytoplasm. They comprise approximately 10 to 20% in normal peripheral blood lymphocytes, 15 to 25% in liver lymphocytes, and 1 to 5% in spleen lymphocytes. Resting NK cells circulate in the blood, but following activation by cytokines, they are capable of extravasation and infiltration into most tissues that contain pathogen-infected or malignant cells (Colucci, F., Di Santo, J.P. and Leibson, P.J., Nat. Immunol., 3, 807, 2002; Kelly J. M., Darcy P. K., Markby J. L., Godfrey D. I., Takeda K., Yagita H., Smyth M. J., Nat. Immunol., 3, 83, 2002; Shi F. D., Wang H. B., Li H., Hong S., Taniguchi M., Link H., Van Kaer L., Ljunggren H. G., Nat. Immunol., 1, 245, 2000; Korsgren M., Persson C. G., Sundler F., Bjerke T., Hansson T., Chambers B. J., Hong S., Van Kaer L., Ljunggren H. G., Korsgren O., J. Exp. Med., 189, 553, 1999). In general, phenotype of NK cells is characterized by the expression of the CD56 and CD 16 (in human), NKR-P1C (NK1.1 in mouse, CD 161 in human), DX5, Ly49 (in mouse; these are restricted to certain mouse strains) surface antigen, and the lack of CD3. The majority (comprising 90% of total NK cells) of human NK cells have low-density expression of CD56 (CD56^{dim}, more cytotoxic) and express high levels of Fcg receptor III (FcgRIII, CD16), whereas ∼10% of NK cells are CD56^{brignt}CD16^{dim} or CD56^{bright}CD16⁻(Schattner, A. and Duggan, D. B., Arthritis Rheum., 27, 1072, 1984). Unlike other immune cells, NK cells can destroy virus-infected cells and tumor cells, etc. in the absence of prior sensitization. NK cells play a central role in early host defense via interaction of activating receptors with their ligands.

NK cells have an ability to discriminate between normal cells and cells lacking the expression of major histocompatibility complex (MHC) class I molecules due to the recognition through NK inhibitory receptors that specific for MHC class I molecules. The functions of NK cells are regulated by a balance between activating receptors and inhibitory receptors that interact with their ligand such as MHC class I or MHC class I-related molecules (non-classical MHC class I) on the target cells (Rajaram, N., Tatake, R. J., Advani, S. H. and Gangal, S. G, Br. J. Cancer, 62, 205, 1990). These receptors are divided into two structural families: immunoglobulin superfamily (leukocyte inhibitory receptors, killer cell Ig-like receptors (KIR, CD158) and C-type lectin-like family (NKG2D, CD94/NKG2, lymphocyte antigen 49 (LY49)). NK cells also produce several cytokines, such as interferon- γ(IFN-γ and tumor-necrosis factor- α(TNF-α, following interaction of cell-surface receptors with their ligand (Bryson, J. S. and Flanagan, D. L., J. Hematother. Stem Cell Res,. 9, 307, 2000).

Whereas effector function of NK cells can be stimulated by cytokines including interleukin-2 (IL-2), IL-12, IL-15, IL-18, IL-21, and type I interferon (IFN-αb) in combination with differential engagement of cell surface receptors, they produce immunoregulatory cytokines such as IFN-γ IL-5, IL-10, IL-13, TNF-α and granulocyte-macrophage colony-stimulating factor (GM-CSF), as well as a number of chemokines following interaction of NK receptors with their ligands (Shi F. D., Wang H. B., Li H., Hong S., Taniguchi M., Link H., Van Kaer L., Ljunggren H. G., Nat. Immunol., 1, 245, 2000). In human, CD56^{bright} NK cell subset also produces several cytokines including IFN-γ, TNF-α TNF-β IL-10 and GM-CSF (Lian R. H., Maeda M., Lohwasser S., Delcommenne M., Nakano T., Vance R. E., Raulet D. H., Takei F., J. Immunol., 168, 4980, 2002), but CD56^{dim} NK cell subset do not produce these cytokines (Colucci, F., Di Santo, J.P. and Leibson, P.J., Nat. Immunol., 3, 807, 2002; Shi F. D., Wang H. B., Li H., Hong S., Taniguchi M., Link H., Van Kaer L., Ljunggren H. G., Nat. Immunol., 1, 245, 2000). Although immature NK cells can produce Th2 cytokines such as IL-5 and IL-13, the ability to produce Th2 cytokines is lost upon terminal development; instead, mature NK cells acquire ability to produce IFN-γ (Van Beneden K., Stevenaert F., De Creus A., Debacker V., De Boever J., Plum J., Leclercq G., J. Immunol., 166, 4302, 2001). NK cells secrete and respond to a number of chemokines including XCL1, CCL1, CCL3, CCL4, CCL5, CCL22, and CXCL8 (Lundwall, A., Dackowski, W., Cohen, E., Shaffer, M., Mahr, A., Dahlback, B., Stenclrclr, J. and Wydro, R., Proc. Natl Acad. Sci., 83, 6716, 1986), which are regulated, in part, by IL-15 (Crosier, K. E. and Crosier, P. S., Pathology, 29, 131, 1997; Nakano, T., Kawamoto, K., Kishino, J., Nomura, K., Higashino, K. and Arita, H., J. Biochem., 323, 387, 1997; Fridell, Y. W., Villa, J., Jr, Attar, E. C. and Liu, E. T., J. Biol. Chem., 273, 7123, 1997), or IL-2 (Goruppi, S., Ruaro, E. and Schneider, C., Oncogene, 12, 471, 1996). These chemokines play an important role in NK cell homing to infected and neoplastic cells in secondary lymphoid tissues, where their production of IFN-γ may serve to directly regulate T cell responses (Lundwall, A., Dackowski, W., Cohen, E., Shaffer, M., Mahr, A., Dahlback, B., Stenclrclr, J. and Wydro, R., Proc. Natl Acad. Sci., 83, 6716, 1986). While resting CD56^{dim}/CD16⁺ NK cell subsets express CXCR1, CXCR2, CXCR3, and CXCR4, CD56^{bright}/CD16⁻ NK cells express high levels of CCR5 and CCR7. Cytolytic activity ofNK cells is stimulated by CCL2, CCL3, CCL4, CCL5, CCL10, and CXC3L1.

There are two different mechanisms known for NK cells to destroy cancer cells. According to the first mechanism, a cellular receptor is utilized. NK cells express three types of tumor necrosis factor protein on cell surface; i.e., FAS ligand (FASL), tumor necrosis factor and TRAIL, all of which are known to bind to their receptors on cancer cells to induce apoptosis of the cancer cells (Ashkenazi, A., Nature Rev. Cancer., 2, 420, 2002). The other mechanism for NK cells to destroy cancer cells is via cytoplasmic granular materials such as perforin or granzyme. These granular materials can make a hole in cellular membrane of cancer cells and consequently destroy the cells by lysis (Trapani, J. A., Davis, J., Sutton, V. R. and Smyth, M. J., Curr. Opin. Immunol., 12, 323, 2000).

Although NK cells have been well known to be derived from pluripotent hematopoietic stem cells (HSCs) (Lian R. H., Maeda M., Lohwasser S., Delcommenne M., Nakano T., Vance R. E., Raulet D. H., Takei F., J. Immunol., 168, 4980, 2002), the ontogeny of NK cells has not been fully understood yet. HSCs (Lin⁻CD34⁺ in human, Lin⁻*c*-*kit*⁺Sca2⁺ in mouse) can be derived from fetal thymus, fetal liver, umbilical cord blood, and have a potential to develop into the common T/NK bipotent progenitors (Lian R. H., Kumar V., Semin. Immunol., 14, 453, 2002; Douagi I., Colucci F., Di Santo JP., Cumano A., Blood, 99, 473, 2002), which are committed to pNK upon *in vitro* culture in combination with IL-7, stem cell factor (SCF), and flt3L (Williams N. S., Klem J., Puzanov I. J., Sivakumar P. V., Bennett M., Kumar V., J. Immunol,. 163, 2648, 1999). Here, differentiation means a phenomenon that a relatively simple system is divided into at least two qualitatively different partial systems. In other words, structure or function becomes specialized. Precursor NK cells, which are intermediate stage cells to mNK cells, are widely distributed among bone marrow, fetal thymus, blood, spleen, and liver, etc. However, incapable of producing interferon-γ, they do not possess a cytolytic activity. In the present invention, a marker for hematopoietic cell is defined as c-kit⁺Lin⁻ (mouse), and CD34⁺ (human).

When pNK cells (CD56-CD122⁺CD34⁺ in human, CD122⁺ NK1.1⁻DX5⁻ in mouse) are subsequently cultured in the presence of IL-15, the cells are able to develop into immature NK cells (CD122⁺CD161-CD56⁻KIR⁻ in human, CD122⁺CD2⁺NK1.1DX⁵⁺Ly49⁻ in mouse). Prolonged culture of pNK cells with IL-15 alone can generate the pseudomature lytic NK cells (CD122⁺CD2⁺NK1.1⁺DX5⁺CD94/NKG2⁺Ly49⁻), which have partial cytolytic activities. Stromal cells provide both various cytokines and substances that can directly contact *via* cell surface receptors to developing NK cells, indicating that they are essential for the generation of lytic LyS49⁺ mNK cells as well as the maturation of NK cells (Iizuka K., Chaplin D. D., Wang Y., Wu Q., Pegg L. E., Yokoyama W. M., Fu Y. X., Proc. Natl. Acad. Sci., 96, 6336, 1999; Briard D., Brouty-Boye D., Azzarone B., Jasmin C., J. Immunol., 168, 4326, 2002). Thus, high frequency of activated lytic Ly49⁺ mNK cells (CD56⁺KIR⁺CD3⁻ in human, CD122⁺CD2⁺NK1.1⁺DX5⁺CD94/NKG2⁺Ly49⁺CD3⁻ in mouse) arises from the co-culture of pNK cells with stromal cells in the presence of IL-15 (Williams N. S., Klem J., Puzanov I. J., Sivakumar P. V., Bennett M., Kumar V., J. Immunol,. 163, 2648, 1999). During differential stages of NK cells during NK cell development, CD94, NKG2A, NKG2C and Ly49B were expressed at the early stages of development, and Ly49G, Ly49C, Ly491 in order, and finally, Ly49A, D, E and F (Williams N. S., Kubota A., Bennett M., Kumar V., Takei F., Eur. J. Immunol., 30, 2074, 2000). In the present invention, a marker for precursor NK cells is defined as CD122⁺NK1.1⁻ (mouse).

pNK cells express transcription factors such as PU.1, GATA3, Id2, and Ets-1. Deficiency of ikaros (Boggs S. S., Trevisan M., Patrene K., Geogopoulos K., Nat. Immunol., 16, 137, 1998), PU.1 (Colucci F., Samson S. I., DeKoter R. P., Lantz O., Singh H., Di Santo J. P., Blood, 97, 2625, 2001), and Id2 (Ikawa T., Fujimoto S., Kawamoto H., Katsura Y, Yokota Y, Proc. Natl. Acad. Sci,. 98, 5164, 2001) causes reduced numbers of pNK cells in mice. In hematopoietic cells, transcription factors such as myeloblastosis (myb) oncogene, c-myc, and Oct 2b are expressed and they play an important role in the regulation of development and proliferation of pNK cells (Bar-Ner M., Messing L. T., Segal S., Immunobiology, 185, 150, 1992; Melotti P., Calabretta B., Blood, 87, 2221, 1996). In pNK cells, immune regulators such as Fc receptor, TNF receptor, IL-7 receptor, chemokine receptor, arid CD36 seem to have critical roles in this stage.

In mNK cells, signaling molecules such as regulator of G protein signaling (RGS), lymphocyte-specific protein tyrosine kinase, and *Fyn* proto-oncogene are known to be involved with NK cell maturation. (Ikawa T., Fujimoto S., Kawamoto H., Katsura Y, Yokota Y, Proc. Natl. Acad. Sci,. 98, 5164, 2001; Ogasawara K., Hida S., Azimi N., Tagaya Y, Sato T., Yokochi-Fukuda T., Waldmann T. A., Taniguchi T., Taki S., Nature, 391, 700, 1998). mNK cells refer to the cells capable of recognizing cancer cells and destroying them directly. It is known that the treatment of mNK cells with IL-2 induces the proliferation and activation of mNK cells. Receptor tyrosine kinases (RTK) constitute a large class of transmembrane proteins that relay extracellular stimuli into intracellular signals for cell proliferation, development, survival and migration (Ullrich, A., Schlessinger, J., Cell, 61, 203, 1990; Fantl, W. J., Johnson, D. E., Williams, L. T., Biochem., 62, 453, 1993; Heldin, C., Cell, 80, 213, 1995). All RTK have a highly conserved cytoplasmic kinase domain that is activated upon growth factor binding to receptor monomers. In the present invention, a marker for mNK cells is defined as CD122⁺NK1⁺ (mouse) and CD34⁺ (human), respectively. Further, a marker for NK cells that are completely matured among mNK cells is defined as Family Ly49A⁺, Ly49C⁺, Ly49D⁺, Ly49E⁺, Ly49F⁺, Ly49G⁺, Ly49H⁺, Ly49I⁺, NKG2A/C/E⁺(mouse), CD56⁺, NKG2A⁺, CD161⁺, NKP46⁺, NKP30⁺, NKP44⁺, and NKG20⁺(human).

RTK activation leads to their auto-phosphorylation, and the tyrosine phosphorylation of multiple downstream intracellular signaling molecules, which are then able to initiate a variety of signal transduction cascades. Axl receptor tyrosine kinase (named also ARK, UFO, or TYRO7) is the first discovered member of a subfamily of RTK that share a unique structure, with extracellular regions composed of two immunoglobulin-related domains linked to two fibronectin type-III repeats, and cytoplasmic regions that contain an intrinsic tyrosine kinase domain (O'Bryan, J. P., Frye, R. A., Cogswell, P.C., Neubauer, A., Kitch, B., Prokop, C., Espinosa, R. III, Lebeau, M. M., Earp, H. S., Liu, E. T., Mol. Cell. Biol., 11, 5016, 1991). Axl is expressed in breast, skeletal muscle, heart, hematopoietic tissue, testis, ovarian follicles and uterine endometrium (Faust, M., Ebensperger C., Schulz, A. S., Schleithoff, L., Hameister, H., Bartram, C. R. and Janssen, J. W., Oncogene, 7, 1287, 1992; Graham, D. K., Bowman G. W., Dawson, T. L., Stanford W. L., Earp, H. S., and Snodgrass, H. R., Oncogen, 10, 2349, 1995; Neubauer, A., Fiebeler, A., Graham, D. K., O'Bryan, J. P., Schmidt, C. A., Barckow, P., Serke, S., Siegert, W., Snodgrass, H. R., Huhn, D., Blood, 84, 1931, 1994; Berclaz, G., Altermatt, H.J., Rohrbach, V., Kieffer, I., Dreffer, E. and Andres, A. C., Ann. Oncol., 12, 819, 2001; Wimmel, A., Glitz, D., Kraus, A., Roeder, J. and Schuermann, M., Eur. J. Cancer., 37, 2264, 2001; Sun, W. S., Misao, R., Iwagaki, S., Fujimoto, J. and Tamaya, T., Mol. Hum. Reprod., 8, 552, 2002). Axl is typified by the cell adhesion molecule-related extracellular ligand-binding domain, composed of two immunoglobulin-like motifs and two fibronectin type III motifs. Recent studies suggest a role of Axl in developmental processes of the hematopoietic and nervous systems, and in tumorigenesis (Crosier, K. E. and Crosier, P. S., Pathology, 29, 131, 1997).

Gas6, the protein product of growth arrest-specific gene 6 (Gas6), is a member of the vitamin K-dependent protein family that was identified as a ligand for the Ax1/Sky family of receptor tyrosine kinases including Axl, Sky (Rse, Brt, Tif, Dtk, Etk-2 and Tyro3) and Mer (c-Eyk, Nyk and Tyro12) (Godowski, P. J., Mark, M. R., Chen, J., Sadick, M. D., Raab, H. and Hammonds R. G, Cell, 82, 355, 1995; Varnum, B. C., Young, C., Elliott, G, Garcia, A., Bartley, T. D., Fridell, Y. W., Hunt, R. W., Trail, G., Clogston, C., Toso, R. J., Nature, 373, 623, 1995; Chen, J., Carey, K. and Godowski, P. J., Oncogene, 14, 2033, 1997). It is known that Gas6 has 46% amino acid identity to protein S, a serum protein that negatively regulates blood coagulation (Manconrftti, G., Brancolini, C., Avanzi, G. and Schneider, C., Mol. Cell. Biol., 13, 4976, 1993). Gas6 is expressed in intestine, testicular somatic cells, pulmonary endothelium and uterine endometrium, and in uterineendometrial cancers (Prieto, A. L., Weber, J. L., Tracy, S., Heeb, M. J. and Lai, C., Brain Res., 816, 646, 1999; Chan, M. C. W., Mather, J. P., Mccray, G and Lee, W. M., J. Androl., 21, 291, 2000; Wimmel, A., Glitz, D., Kraus, A., Roeder, J. and Schuermann, M., Eur. J. Cancer., 37,2264,2001).

The production of Gas6 with full biological activity requires a vitamin K-dependent post-translational modification in which the newly synthesized peptide is extensively γ-carboxylated in the endoplasmic reticulum (Manconrftti, G, Brancolini, C., Avanzi, G. and Schneider, C., Mol. Cell. Biol., 13, 4976, 1993; Lundwall, A., Dackowski, W., Cohen, E., Shaffer, M., Mahr, A., Dahlback, B., Stenclrclr, J. and Wydro, R., Proc. Natl Acad. Sci., 83, 6716, 1986; Chen, J., Carey, K. and Godowski, P. J., Oncogene, 14, 2033, 1997). Gas6 acts as a growth-potentiating factor for thrombin-induced proliferation of vascular smooth muscle cells (Nakano, T., Kawamoto, K., Kishino, J., Nomura, K., Higashino, K. and Arita, H., J. Biochem., 323, 387, 11997). Additionally, Gas6 has been shown to be a novel chemoattractant that induces Axl-mediated migration of vascular smooth muscle cells (Davis, J. E., Smyth, M. J. and Trapani, J. A., Eur. J. Immunol., 31, 39-47, 2001). Gas6 is also able to induce cell cycle re-entry and protect serum-starved NIH3T3 cells from apoptotic cell death (Goruppi, S., Ruaro, E. and Schneider, C., Oncogene, 12, 471, 1996). Recently, Gas6 has been shown to induce beta-catenin stabilization and T-cell factor transcriptional activation in mammary cells (Goruppi, S., Chiaruttini, C., Ruaro, M. E., Varnum, B. and Schneider, C., Mol. Cell. Biol., 21, 902, 2001).

Protein S is a vitamin-K dependent plasma glycoprotein that serves as an important cofactor for activated protein C in the blood anticoagulation system. Protein S also acts as a mitogen on distinct cell types and is a ligand for Tyro3, a member of the Axl family of oncogenic receptor tyrosine kinases (Wimmel A. et al., Cancer1999 Jul 1;86(1):43-9). It has been known that Protein S stimulates a member of Axl/Sky protein, similar to Gas 6. Protein S has a similar structure to Gas 6 (i.e., it is constituted with Gla domain at N-terminus, four EGF-like domain, and G domain like a signal transduction molecule) and can phosphorylate Axl/Sky protein family (Evenas P, et al., Biol. Chem. 2000 Mar;381(3):199-209).

Protein S exists in two forms in human plasma, namely as the free protein and in complex with C4b-binding protein (Dahlback & Stenflo (1981) Proc. Nat1. Acad. Sci. U.S.A. 78, 2512-2516). Protein S can be isolated by several simple purification methods, which include for example, barium citrate adsorption, DEAE-Sephacel chromatography and chromatography on Blue Sepharose (Dahlback B., Biochem. J. 1983 Mar 1;209(3):837-846). In addition, Protein S can be produced by genetic recombination method (Merel Van Wijnen, et al., Biochem. J. 330, 389-396).

Recombinant DNA methods used herein are generally, those set forth in Sambrook et al. (Molecular Cloning: A Laboratory Manual, Cold Spring Harbor Laboratory Press, Cold Spring Harbor, N.Y. (1989)) and/or Ausubel et al., eds., (Current Protocols in Molecular Biology, Green Publishers Inc. and Wiley and Sons, N.Y (1994)). For example, by inserting a nucleic acid sequence which encodes the amino acid sequence of Axl, Gas6 or protein S polypeptide into an appropriate vector, one skilled in the art can readily produce large quantities of the desired nucleotide sequence. The sequences can then be used to generate detection probes or amplification primers. Alternatively, a polynucleotide encoding the amino acid sequence of Axl, Gas6 or protein S polypeptide can be inserted into an expression vector. By introducing the expression vector into an appropriate host, the encoded Axl, Gas6 or protein S polypeptide may be produced in large amounts. Another method for obtaining a suitable nucleic acid sequence is the polymerase chain reaction (PCR). In this method, cDNA is prepared from poly(A)+RNA or total RNA using the enzyme reverse transcriptase. Two primers, typically complementary to two separate regions of cDNA (oligonucleotides) encoding the amino acid sequence of Axl, Gas6 or protein S polypeptide, are then added to the cDNA along with a polymerase such as Taq polymerase, and the polymerase amplifies the cDNA region between the two primers. A nucleic acid molecule encoding the amino acid sequence of Axl, Gas6 or protein S polypeptide may be amplified/expressed in prokaryotic, yeast, insect (baculovirus systems), and/or eukaryotic host cells (Meth. Enz. vol. 185 D. V. Goeddel ed., Academic Press, Sna Diego Calif., 1990). Typically, expression vectors used in any of the host cells will contain sequences for plasmid maintenance and for cloning arid expression of exogenous nucleotide sequences. Such sequences include a promoter, an enhancer sequence, an origin of replication, a transcriptional termination sequence, a sequence encoding a leader sequence for secretion, a ribosome binding site, a polyadenylation sequence, a polylinker region for inserting the nucleic acid encoding the polypeptide to be expressed, and a selectable marker element,etc. Such flanking sequences are all well known to a skilled person in the pertinent art and can be easily selected by him.

Examples of suitable promoters for directing the transcription of the DNA encoding the human Axl, Gas6 or protein S in mammalian cells are the SV40 promoter (Subramani et al., Mol. Cell Biol. 1 (1981), 854-864), the MT-1 (metallothionein gene) promoter (Palmiter et al., Science 222 (1983), 809-814), the CMV promoter (Boshart et al., Cell 41:521-530, 1985) or the adenovirus 2 major late promoter (Kaufman and Sharp, Mol. Cell. Biol, 2:1304-1319, 1982). An example of a suitable promoter for use in insect cells is the polyhedrin promoter (U.S. Pat. No. 4,745,051; Vasuvedan et al., FEBS Lett. 311, (1992) 7-11), the P10 promoter (J. M. Vlak et al., J. Gen. Virology 69, 1988, pp. 765-776), the baculovirus immediate early gene 1 promoter (U.S. Pat. No. 5,155,037; U.S. Pat. No. 5,162,222), or the baculovirus 39K delayed-early gene promoter (U.S. Pat. No. 5,155,037; U.S. Pat. No. 5,162,222). Examples of suitable promoters for use in yeast host cells include promoters from yeast glycolytic genes (Hitzeman et al., J. Biol. Chem. 255 (1980), 12073-12080; Alber and Kawasaki, J. Mol. Appl. Gen. 1 (1982), 419-434) or alcohol dehydrogenase genes (Young et al., in Genetic Engineering of Microorganisms for Chemicals (Hollaender et. al, eds.), Plenum Press, New York, 1982), or the TPI1 (U.S. Pat. No. 4,599,311) or ADH2-4c (Russell et al., Nature 304 (1983), 652-654) promoters. Examples of suitable promoters for use in filamentous fungus host cells are, for instance, the ADH3 promoter (McKnight et al., The EMBO J. 4 (1985), 2093-2099) or the tpiA promoter. Examples of other useful promoters are those derived from the gene encoding A olyzae TAKA amylase, Rhizomucor miehei aspartic proteinase, A. niger neutral.alpha.-amylase, A. niger acid stable o-amylase, A. niger or A. awamori glucoamylase (gluA), Rhizomucor miehei lipase, A. oryzae alkaline protease, A. oryzae triose phosphate isomerase or A. nidulans acetamidase.

The DNA sequences may also, if necessary, be operably connected to a suitable terminator, such as the human growth hormone terminator (Palmiter et al., Science 222, 1983, pp. 809-814) or the TPI1 (Alber and Kawasaki, J. Mol. App1. Gen. 1,1982, pp. 419-434) or ADH3 (McKnight et al., The EMBO J. 4,1985, pp. 2093-2099) terminators. Expression vectors may also contain a set of RNA splice sites located downstream from the promoter and upstream from the insertion site for the DNA sequence itself. Preferred RNA splice sites may be obtained from adenovirus and/or immunoglobulin genes. Also contained in the expression vectors is a polyadenylation signal located downstream of the insertion site. Particularly preferred polyadenylation signals include the early or late polyadenylation signal from SV40, the polyadenylation signal from the adenovirus 5 E1b region or the human growth hormone gene terminator (DeNoto et al. Nucl. Acids Res. 9:37193730, 1981). The expression vectors may also include a noncoding viral leader sequence, such as the adenovirus 2 tripartite leader, located between the promoter and the RNA splice sites; and enhancer sequences, such as the SV40 enhancer.

To direct the human polypeptide of the present invention into the secretory pathway of the host cells, a secretory signal sequence (also known as a leader sequence, prepro sequence or pre sequence) may be provided in the recombinant vector. The secretory signal sequence is joined to the DNA sequences encoding the human polypeptide in the correct reading frame. Secretory signal sequences are commonly positioned 5' to the DNA sequence encoding the peptide. The secretory signal sequence may be that, normally associated with the protein or may be from a gene encoding another secreted protein. For secretion from yeast cells, the secretory signal sequence may encode any signal peptide, which ensures efficient direction of the expressed human polypeptide into the secretory pathway of the cell. The signal peptide may be naturally occurring signal peptide, or a functional part thereof, or it may be a synthetic peptide. Suitable signal peptides have been found to be the α -factor signal peptide (U.S. Pat. No. 4,870,008), the signal peptide of mouse salivary amylase (O. Hagenbuchle et al., Nature 289,1981, pp. 643646), a modified, carboxypeptidase signal peptide (L. A. Valls et al., Cell 48, 1987, pp. 887-897), the yeast BAR1 signal peptide (WO 87/02670), or the yeast aspartic protease 3 (YAP3) signal peptide (M. Ege1-Mitani et al., Yeast 6, 1990, pp. 127-137). For efficient secretion in yeast, a sequence encoding a leader peptide may also be inserted downstream of the signal sequence and upstream of the DNA sequence encoding the human polypeptide. The function of the leader peptide is to allow the expressed peptide to be directed from the endoplasmic reticulum to the Golgi apparatus and further to a secretory vesicle for secretion into the culture medium (i.e. exportation of the human polypeptide across the cell wall or at least through the cellular membrane into the periplasmic space of the yeast cell). The leader peptide may be the yeast alpha-factor leader (the use of which is described in e.g. U.S. Pat. No. 4,546,082, U.S. Pat. No. 4,870,008, EP 16 201, EP 123 294, EP 123 544 and EP 163 529). Alternatively, the leader peptide may be a synthetic leader peptide, which is to say a leader peptide not found in nature. Synthetic leader peptides may, for instance, be constructed as described in WO 89/02463 or WO 92/11378. For use in filamentous fungi, the signal peptide may conveniently be derived from a gene encoding an Aspergillus sp. amylase or glucoamylase, a gene encoding a Rhizomucor miehei lipase or protease or a Humicola lanuginosa lipase. For use in insect cells, the signal peptide may conveniently be derived from an insect gene (WO 90/05783), such as the lepidopteran Manduca sexta adipokinetic hormone precursor signal peptide (U.S. Pat. No. 5,023,328).

Methods of transfecting mammalian cells and expressing DNA sequences introduced in the cells are described in e.g. Kaufman and Sharp, J. Mol. Biol 159 (1982), 601-621; Southern and Berg, J. Mol. Appl. Genet 1 (1982), 327-341; Loyter et al., Proc. Natl. Acad. Sci. USA 79 (1982), 422-426; Wigler et al., Cell 14 (1978), 725; Corsaro and Pearson, Somatic Cell Genetics 7 (1981), 603, Graham and van der Eb, Virology 52 (1973), 456; and Neumann et al., EMBO J. 1 (1982), 841-845. Cloned DNA sequences are introduced into cultured mammalian cells by, for example, calcium phosphate-mediated transfection (Wigler et al., Cell 14:725-732,1978; Corsaro and Pearson, Somatic Cell Genetics 7:603-616, 1981; Graham and Van der Eb, Virology 52d:456467, 1973) or electroporation (Neumann et al., EMBO J. 1:841-845, 1982). To identify and select cells that express the exogenous DNA, a gene that confers a selectable phenotype (a selectable marker) is generally introduced into cells along with the gene or cDNA of interest. Preferred selectable markers include genes that confer resistance to drugs such as neomycin, hygromycin, and methotrexate. The selectable marker may be an amplifiable selectable marker. A preferred amplifiable selectable marker is a dihydrofolate reductase (DHFR) sequence. Selectable markers may be introduced into the cell on a separate plasmid at the same time as the gene of interest, or they may be introduced on the same plasmid. If, on the same plasmid, the selectable marker and the gene of interest may be under the control of different promoters or the same promoter, the latter arrangement producing a dicistronic message. Constructs of this type are known in the art (for example, U.S. Pat. No. 4,713,339). It may also be advantageous to add additional DNA, known as "carrier DNA," to the mixture that is introduced into the cells.

After the cells have taken up the DNA, they are grown in an appropriate growth medium, typically 1-2 days, to begin expressing the gene of interest. As used herein the term "appropriate growth medium" means a medium containing nutrients and other components required for the growth of cells and the expression of the human polypeptide of interest. Media generally include a carbon source, a nitrogen source, essential amino acids, essential sugars, vitamins, salts, phospholipids, protein and growth factors. For production of γ-carboxylated proteins, the medium will contain vitamin K, preferably at a concentration of about 0.1 µg/ml to about 5 µg/ml. Drug selection is then applied to select for the growth of cells that are expressing the selectable marker in a stable fashion. For cells that have been transfected with an amplifiable selectable marker the drug concentration may be increased to select for an increased copy number of the cloned sequences, thereby increasing expression levels. Clones of stably transfected cells are then screened for expression of the human polypeptide of interest.

The host cell into which the DNA sequences encoding the human polypeptide of interest is introduced may be any cell, which is capable of producing the posttranslational modified human polypeptide and includes yeast, fungi and higher eukaryotic cells. Examples of mammalian cell lines for use in the present invention are the COS-1 (ATCC CRL 1650), baby hamster kidney (BHK) and 293 (ATCC CRL 1573; Graham et al., J. Gen. Virol. 36:59-72, 1977) cell lines. A preferred BHK cell line is the tk⁻ ts13 BHK cell line (Waechter and Baserga, Proc. Natl. Acad. Sci. USA 79:1106-1110, 1982). In addition, a number of other cell lines may be used within the present invention, including Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1), CHO (ATCC CCL 61) and DUKX cells (Urlaub and Chasin, Proc. Natl. Acad. Sci. USA 77:4216-4220,1980). Examples of suitable yeasts cells include cells of Saccharomyces spp. or Schizosaccharomyces spp., in particular strains of Saccharomyces cerevisiae or Saccharomyces kluyveri. Methods for transforming yeast cells with heterologous DNA and producing heterologous polypeptides therefrom are described, e.g. in U.S. Pat. No. 4,599,311, U.S. Pat. No. 4,870,008, 5,037,743, and U.S. Pat. No. 4,845,075. Transformed cells are selected by a phenotype determined by a selectable marker, commonly drug resistance or the ability to grow in the absence of a particular nutrient, e.g. leucine. A preferred vector for use in yeast is the POT 1 vector disclosed in U.S. Pat. No. 4,931,373. Examples of other fungal cells are cells of filamentous fungi, e.g. Aspergillus spp., Neurospora spp., Fusarium 6 pp. or Trichoderma spp., in particular strains of A. oryzae, A. nidulans or A. niger. The use of Aspergillus spp. for the expression of proteins is described in, e.g., EP 272 277, EP 238 023, EP 184 438. The transformation of F. oxysporum may, for instance, be carried out as described by Malardier et al., 1989, Gene 78: 147-156. The transformation of Trichoderma spp. may be performed for instance as described in EP 244 234. When a filamentous fungus is used as the host cell, it may be transformed with the DNA construct of the invention, conveniently by integrating the DNA construct in the host chromosome to obtain a recombinant host cell. This integration is generally considered to be an advantage as the DNA sequence is more likely to be stably maintained in the cell. Integration of the DNA constructs into the host chromosome may be performed according to conventional methods, e.g. by homologous or heterologous recombination. Transformation of insect cells and production of heterologous polypeptides therein may be performed as described in U.S. Pat. No. 4,745,051; U.S. Pat. No. 4,879,236; U.S. Pat. Nos. 5,155,037; 5,162,222; EP 397,485. The insect cell line used as the host may suitably be a Lepidoptera cell line, such as Spodoptera frugiperda cells or Trichoplusia ni cells (U.S. Pat. No. 5,077,214). Culture conditions may suitably be as described in, for instance, WO 89/01029 or WO 89/01028.

The transformed or transfected host cell described above is then cultured in a suitable nutrient medium under conditions permitting expression of the human polypeptide after which all or part of the resulting peptide may be recovered from the culture. The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The proteins produced by the cells may then be recovered from the culture medium by, conventional procedures including separating the host cells from the medium by centrifugation or filtration, precipitating the aqueous protein components of the supernatant or filtrate by means of a salt, e.g. ammonium sulphate, purification by a variety of chromatographic procedures, e.g. ion exchange chromatography, gel filtration chromatography, affinity chromatography, or the like, dependent on the type of polypeptide in question.

Axl antibody used according to the present invention can be either polyclonal or monoclonal. Axl antibody can be purchased from Santa Cruz Biotech. The antibody used in the present invention can be prepared based on a publicly known method.

Polyclonal antibody is obtained by subcutaneous or peritoneal injection of an antigen and an adjuvant to an animal. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester, N-hydroxysuccinimide, glutaraldehyde, succinic anhydride or SOCl₂. Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to fourteen days later the animals are bled and the serum is assayed for antibody titer. Animals are boosted until the titer plateaus. Preferably, the animal is boosted with the conjugate of the same antigen, but conjugated to a different protein and/or through a different cross-linking reagent can be also used. Conjugates also can be made in recombinant cell culture as a fusion protein. Also, aggregating agents such as alum are suitable used to enhance the immune response.

Monoclonal antibodies are obtained from a population of substantially homogeneous antibodies, i.e., the individual antibodies comprising the population are identical except for possible naturally occurring mutations that may be present in minor amounts. Thus, the "monoclonal" indicates the character of the antibody as not being a mixture of discrete antibodies, but being able to specifically recognize a single antigen.

For example, the monoclonal antibodies may be prepared using the hybridoma method first described by Kohler et al., Nature, 256:495 (1975), or may be-prepared by recombinant DNA methods (U.S. Pat. No. 4,816,567). In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp. 59 103 (Academic Press, 1986). The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium). These substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, Calif. USA, and SP-2 or X63-Ag8-653 cells available from the American Type Culture Collection, Rockville, Md. USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51 63 (Marcel Dekker, Inc., New York, 1987)). Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The binding affinity of the monoclonal antibody can, for example, be determined by the Scatchard analysis of Munson et al., Anal, Biochem., 107:220 (1980). After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59 103 (Academic Press, 1986)). Suitable culture media for this purpose include, for example, D-MEM or RPMI-1640 medium. In addition, the hybridoma cells may be grown in vivo as ascites tumors in an animal. The monoclonal antibodies secreted by the subclones are suitably separated from the culture medium, ascites fluid, or serum by conventional antibody purification procedures such as, for example, protein A-Sepharose, hydroxylapatite chromatography, gel electrophoresis, dialysis, or affinity chromatography.

Hematopoietic stem cells (HSCs), with capacity to renew themselves, are the source of committed progenitor cells throughout the lifetime of an individual. A general feature of primitive as well as more committed hematopoietic cells is the expression of the CD34 antigen, which can be detected by FACS (fluorescence-activated cell sorter) analysis using anti-CD34 monoclonal antibodies. HSC is derived from bone marrow, peripheral blood, umbilical cord blood, etc. Umbilical cord blood (UCB) is known to be a rich source of hematopoietic stem cells (SCs). UCB, obtained from the placenta directly after delivery, is enriched in SCs and has a higher proliferative capacity than cells obtained from bone marrow and peripheral blood.. The introduction of hematopoietic growth factors such as G-CSF has greatly facilitated the mobilization of CD34(+) cells (Beyer J et al., Hematopoietic rescue after high-dose chemotherapy using autologous peripheral-blood progenitor cells or bone marrow: a randomized comparison. J Clin Oncol 1995;13:1328-1335; and Smith TJ et al., Economic analysis of a randomized clinical trial to compare filgrastim-mobilized peripheral-blood progenitor-cell transplantation and autologous bone marrow transplantation in patients with Hodgkin's and non-Hodgkin's lymphoma. J Clin Oncol 1997;15:5-10). G-CSF is administered in dosage of 300-960 µg/d until the end of the collection period. Strategies employed to isolate or purify such cells are numerous, and these include sorting of cells according to specific cell surface markers using either fluorescence (Preffer FI, et al., Lineage-negative side-population(SP) cells with restricted hematopoietic capacity circulate in normal human adult blood: immunophenotypic and functional characterization. STEM CELLS 2002; 20:417427) or immunomagnetic technology (Przyborski SA. Isolation of human embryonal carcinoma stem cells by immunomagnetic sorting. Stem Cells 2001; 19:500504), exploiting the differential plating efficiencies of stem cells on culture plastic (Friedenstein AJ, et al., Fibroblast precursors in normal and irradiated mouse hematopoietic organs. Exp Hematol 1976; 4:267274), and column-separation techniques (Huss R. Isolation of primary and immortalized CD34 hematopoietic and Mesenchymal stem cells from various sources. STEMCELLS 2000; 18:19).

It is well known to use IL-2 to activate mNK cells. Two different methods are suggested for the treatment with IL-2. First, IL-2 is directly administered to a patient so that NK cells are proliferated and activated by IL-2 in the patient's own body. Second, blood is drawn from the patient and mNK cells are isolated from the blood and activated by IL-2, and then the resulting activated mNK cells are introduced back to the patient to be treated. Both of these methods aim to destroy cancer cells by NK cells that have been activated by IL-2. However, these methods involve using high dose of IL-2 (about 150ng/ml), which can bring an adverse effect. Specifically, high toxicity, fever, pulmonary edema, and a shock may occur, because IL-2 causes T-lymphocyte to stimulate tumor necrosis factors or other cytokines such as IL-γ so that the cytokines start interacting with vascular endothelial cells and other cells. In order to solve such problems, studies are being carried out for using low dose of IL-2 but a satisfactory result is yet to be found (M.J. Smyth, Y. Hayakawa, K. Takeda, H. Yagita, Nat Rev Cancer. 2,850, 2002; M. A. Caligiuri, et al., J. Exp. Med. 171, 1509,1990).

According to the present invention, it is found that a low dose of about 8 to 15ng/ml of IL-2 is enough for full stimulation and activation of differentiated mNK cells. Preferred dose of IL-2 is about 10 ng/ml. Such low dose of IL-2 appears not to induce any toxicity from activated mNK cells.

Several methods are used for the preservation of cells, of which the best known is cryopreservation. In addition, HypoThermosol (BioLife Solutions Inc) family or dimethyl sulfoxide (DMSO) solutions can be used.

According to an additional aspect of the present invention, NK cells can be preserved at ultra low temperature before and after the administration to a patient. A typical method for the preservation at ultra low temperature in small scale is described in USP 6,0168,991. For small-scale preservation at ultra low temperature, cells can be re-suspended with concentration of 200x10⁶/ml in 5% human albumin serum (HAS) which is previously cooled. Next, an equivalent amount of 20% DMSO is added into said HAS solution. Then, aliquots of the mixture are taken into 1ml vial and frozen overnight inside the ultra low temperature chamber (NalgeneTM) at -80°C. In case of ultra low temperature preservation in large scale, cells can be re-suspended 600x10⁶/ml. in AIM V. Subsequently, the same amount of 20% AIM V is added gradually into the resuspension. The resulting mixture is frozen inside freezer vessel (Cryocyte, Baxter) using speed-controlled freezing system (FormaTM). Effective amount of activated mNK cells for cytotoxicity can be varied depending on use of the cells inside test tube or living human body, as well as the amount and type of cells which are ultimate target of the activated mNK cells.

As used herein, effective amount for cytotoxicity is defined as amount of mNK cell cells that is required for destroying cancer cells (or, being able to cause a pharmaceutical action). Since such effective amount can vary depending on health and severeness of a patient, it should be determined by a physician after considering all of such variables. Generally, 10⁶ to 10¹² cells, preferably 10⁸ to 10¹¹ cells, more preferably 10⁹ to 10¹⁰ cells are administered per each administration to an adult cancer patient. mNK cells proliferated by the method according to the present invention can be administered to a patient subcutaneously, intramuscular, intravenous, and epidurally, together with a pharmaceutically acceptable vehicle (for example, saline solution) for the treatment. Use of various bio materials (cell carrier) can increase the efficiency of mNK cells to be delivered to a target site and to destroy cancer cells. Cell carrier includes methylcellulose of polysaccharides (M.C. Tate, D.A. Shear, S.W. Hoffman, D.G Stein, M.C. LaPlaca, Biomaterials 22, 1113, 2001), chitosan (Suh JKF, Matthew HWT. Biomaterials, 21, 2589, 2000; Lahiji A, Sohrabi A, Hungerford DS, et al., J Biomed Mater Res, 51, 586, 2000), N- isopropylacrylamide copolymer P(NIPAM-co-AA) (Y.H. Bae, B. Vernon, C.K. Han, S.W. Kim, J. Control. Release 53, 249,1998 H. Gappa, M. Baudys, J.J. Koh, S.W. Kim, YH. Bae, Tissue Eng. 7, 35, 2001), as well as Poly(oxyethylene)/poly(D,L-lactic acid-co-glycolic acid) (B. Jeong, K.M. Lee, A. Gutowska, Y.H. An, Biomacromolecules 3, 865, 2002), P(PF-co-EG) (Suggs LJ, Mikos AG. Cell Trans, 8, 345, 1999), PEO/PEG (Mann BK, Gobin AS, Tsai AT, Schmedlen RH, West JL., Biomaterials, 22, 3045, 2001 Bryant SJ, Anseth KS. Biomaterials, 22, 619, 2001), PVA (Chih-Ta Lee, Po-Han Kung and Yu-Der Lee, Carbohydrate Polymers, 61, 348, 2005), collagen (Lee CR, Grodzinsky AJ, Spector M., Biomaterials 22, 3145, 2001), alginate (Bouhadir KH, Lee KY, Alsberg E, Damm KL, Anderson KW, Mooney DJ. Biotech Prog 17, 945, 2001 Smidsrd O, Skjak-Braek G, Trends Biotech, 8, 71, 1990), etc. and they are used as a cell carrier for cellular treatment in the field of tissue engineering.

### Brief Description of the Drawings

Fig. 1 shows a flow diagram of isolation and differentiation of murine bone marrow -derived hematopoietic stem cells into mature natural killer cells (BM: bone marrow; HSC: hematopoietic stem cells; pNK: precursor natural killer cells; mNK: mature natural killer cells).
Fig. 2 shows purity data of differentiation-staged cells by FACS which were developed from HSCs isolated from murine BM according to the prior art method into mNK cells.
Fig. 3 shows an electrophoretic analysis following RT-PCR of specific gene expression in murine BM-derived HSCs, pNK cells differentiated from HSCs, and mNK cells developed from the pNK cells in the absence and presence of OP9 stromal cells.
Fig. 4 shows purity data of mNK cells by FACS which were differentiated by culturing pNK cells either alone or in co-culture with OP9 stromal cells.
Fig. 5 shows the schematic diagram of the steps in SAGE of differentiation-staged cells during the differentiation from HSCs to mNK cells.
Fig. 6 shows the comparison of electrophoresis and SAGE analyses of Axl in differentiation-staged cells during the differentiation from murine HSCs to mNK cells, both being consistent.
Fig. 7 shows the effect of polyclonal antibody directed against Axl according to the present invention on the differentiation from murine pNK cells to mNK cells, analyzed by FACS.
Fig. 8 shows the effect of polyclonal antibody directed against Axl according to the present invention on the differentiation from murine pNK cells to mNK cells, analyzed by electrophoresis following RT-PCR.
Fig. 9 shows the effect of polyclonal antibody directed against Axl according to the present invention and low concentration of IL-15 in the absence of stromal cells on the differentiation from murine pNK cells to mNK cells, analyzed by FACS.
Fig. 10 shows the effect of polyclonal antibody directed against Axl according to the present invention and low concentration of IL-15 in the absence of stromal cells on the differentiation from murine pNK cells to mNK cells, analyzed by electrophoresis following RT-PCR.
Fig: 11 shows the effect of polyclonal antibody directed against Axl according to the present invention on the production of interferon-γ by mNK cells.
Fig. 12 shows the effect of polyclonal antibody directed against Axl according to the present invention on the proliferation of pNK cells.
Fig: 13 shows the effect of murine recombinant Gas6 according to the present invention on the differentiation of murine NK cells.
Fig. 14 shows the effect of warfarin on the differentiation of murine NK cells.
Fig. 15 shows the electrophoretic analysis of the recombinant vector containing cloned murine Gas6 cDNA according to the present invention.
Fig. 16 shows the electrophoretic analysis of the recombinant murine Gas6 expression vector of the present invention.
Fig. 17 shows the electrophoretic analysis of the cloning of murine Gas6 cDNA in the constructed expression vector.
Fig. 18 shows the electrophoretic analysis of the Gas6 expression in murine Gas6 transfectant according to the present invention.
Fig. 19 shows the effect of the murine Gas6 transfectant according to the present invention on the differentiation from murine pNK cells to mNK cells, analyzed by FACS.
Fig. 20 shows the effect of the murine Gas6 transfectant according to the present invention on the differentiation from murine pNK cells to mNK cells, analyzed by electrophoresis following RT-PCR.
Fig. 21 shows the effect of the murine Gas6 transfectant according to the present invention on the production of interferon-γ by mNK cells.
Fig. 22 shows the effect of the murine native Gas6 on the proliferation of pNK cells.
Fig. 23 shows the electrophoretic analysis of the cloning of murine Ax1 cDNA and the direction of the murine Axl cDNA cloned in retrovirus vector pLXSN.
Fig. 24 shows the electrophoretic analysis of the murine Axl-Fc expression vector according to the present invention.
Fig. 25 shows the effect of the murine Axl-Fc fusion protein according to the present invention on the differentiation of NK cells.
Fig. 26 shows the vector system of the present invention based on self-inactivating Lenti virus.
Fig. 27 shows the construction of double-promoter siRNA cassette and siRNA template oligomer used in the present invention.
Fig. 28 shows the electrophoretic analysis of the cloning of Axl siRNA according to the present invention.
Fig. 29 shows the fluorescence and FACS analyses of the level of infection by pFIV-U6/H1-GFP virus in 293T cells.
Fig. 30 shows the FACS analysis of the level of infection by pFIV-U6/H1-GFP virus in murine HSCs and pNK cells.
Fig. 31 shows the effect of Axl siRNA according to the present invention on the differentiation of murine mNK cells, analyzed by FACS.

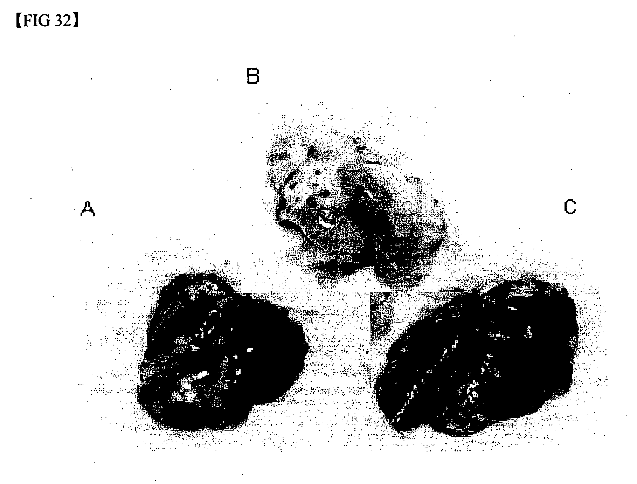

Fig. 32 shows the tumorigenesis-inhibiting effect of NK cells differentiated by Axl polyclonal antibody according to the present invention in an animal model.
Fig. 33 shows the cancer cell-killing effect of NK cells differentiated by Axl polyclonal antibody according to the present invention in an animal model.
Fig. 34 shows survival rate of mouse with induced cancer to which NK cells differentiated by Axl polyclonal antibody according to the present invention were injected.
Fig. 35 shows a recombinant expression vector pET-hAxl/ECD of the present invention producing human Axl protein.
Fig. 36 shows a recombinant expression vector phGas6 of the present invention producing human Gas6 protein.
Fig. 37 shows purity data of mNK cells by FACS which were obtained by separating HSCs from human umbilical cord blood, differentiating them into pNK cells by treating with SCF, Flt3-L, and IL-7, and then differentiating the resulting pNK cells into mNK cells by treating with IL-15.
Fig. 38 shows expression data for perforin and granzyme of the mNK cells analyzed by electrophoresis following RT-PCR, which were obtained by separating HSCs from human umbilical cord blood, differentiating them into pNK cells by treating with SCF, Flt3-L, and IL-7, and differentiating the resulting pNK cells into mNK cells by treating with IL-15.
Fig. 39 shows the cancer cell-killing effect of mNK cells which were obtained by separating HSCs from human umbilical cord blood, differentiating them into pNK cells by treating with SCF, Flt3-L, and IL-7, and then differentiating the resulting pNK cells into mNK cells by treating with IL-15 in cell level experiment.
Fig. 40 shows purity data of mNK cells which were obtained by separating HSCs from human umbilical cord blood, differentiating them into pNK cells, and differentiating the resulting pNK cells into mNK cells by treating with a polyclonal antibody against Ax1 protein, analyzed by FACS.
Fig. 41 shows purity data of mNK cells which were obtained by separating HSCs from human peripheral blood, differentiating them into pNK cells, and differentiating the resulting pNK cells into mNK cells by treating with a polyclonal antibody directed against Axl protein, analyzed by FACS.

### Best Mode

The following examples and drawings are provided for illustration of the invention. They should not be considered as limiting the scope of the invention, but merely as being representative thereof.

### Example 1

### Discovery of specific genes to be expressed during the development of murine natural killer cells

### (A) Isolation of hematopoietic stem cells from murine bone marrow and differentiation into natural killer cells

Whole bones of 8-12 year old mouse (C57BL/6 purchased from Korea Research Bioscience and Biotechnology) were procured under the germ free condition according to animal care and handling guidelines. Bone marrow cells were isolated from those bones and treated with cell lysis buffer (0.2% NaCl, 1.6% NaCl) to remove red blood cells. The remaining cells were reacted with 2.4G2 supernatant over an ice bath and washed with phosphate-buffered solution containing 2 mM EDTA (buffer A). The cells were suspended in buffer A at the concentration of 1 x 10⁸/500 µℓ. The resulting suspension was reacted with a biotin-conjugated antibody cocktail (Mac-1, Gr-1, B220, NK 1.1, CD2, TER-119, Pharmingen) at the temperature of 4°C for 10 minutes. The cells were washed with buffer A. 1 x 10⁸ cells were reacted with 900 µℓ of buffer A and 100 µℓ of streptavidin-microbeads (Miltenyi Biotec) at the temperature of 4°C for 15 minutes. After washing, the cells were suspended in MACS (magnetic bead-activated cell sorting) buffer solution (phosphate-buffered solution containing 2 mM EDTA and 0.5% BSA) and filtered over nylon mesh (70 µm). Magnetic column (CS column, Miltenyi Biotech) was fixed onto super MACS and magnetic bead-labeled cells were passed. The column was sufficiently washed with MACS buffer and the eluted solution was centrifuged to afford lineage negative (Lin⁻) cells. FITC-conjugated anti-c-kit (Pharmingen) was added to Lin⁻ cells (1 x 10⁷) and the reaction was allowed at the temperature of 4°C for 10 minutes. After washing, Lin⁻ cells were again reacted with anti-FITC microbeads (Miltenyi Biotec) at the temperature of 4 °C for 15 minutes and suspended in 500 µℓ of MACS buffer. The cell suspension was passed through an MS column housed within a magnetic field. The column was sufficiently washed with 1 mℓ of MACS buffer to elute c-kit+ cells which were used as hematopoietic cells. The isolated hematopoietic cells were cultured for 6 days in 24-well plate at the concentration of 1 x 10⁶/mℓ while half of RPMI (Gibco) medium containing SCF (30 ng/mℓ, Biosource), IL-7 (0.5 ng/mℓ, PeproTech), Flt31 (50 ng/mℓ, PeproTech), indometacine (2 µg/mℓ, Sigma), and gentamicin ((2 µg/mℓ, Sigma) was replaced with fresh medium every three days. After 6-day culture with said cytokines, the cultured cells were reacted with CD122-FITC antibody and multisort microbeads and MACS was carried out to afford CD122⁺ precursor natural killer (pNK) cells. The pNK cells were then differentiated into mature natural killer cells for 6 days by two ways using RPMI containing IL-15 (20 ng/ mℓ), indometacine (2 µg/mℓ), and gentamicin (2 µg/mℓ). One way for the differentiation into mature natural killer cells (mNK-2) was performed by co-culturing with stromal cells (ATCC). The other way was done without co-culture to afford less mature natural killer cells (mNK-1). These NK cells were collected to analyze the expression of cell surface markers (Fig 1).

### (B) Purity of stage-separated cells during the differentiation from murine hematopoietic stem cells to mature natural killer cells

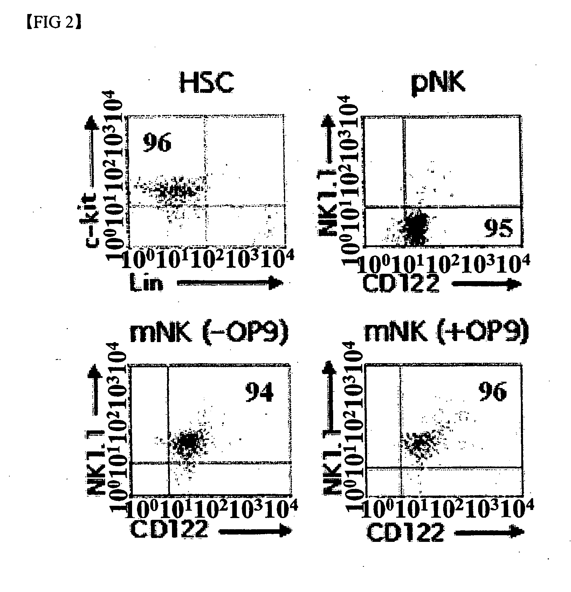

As described in the above Example 1 (A), whole 2 x 10⁸ bone marrow cells were obtained from eight 8-12 year old mice (C57BL/6) and Lin-, c-kit⁺ hematopoietic stem cells were separated. CD122⁺ pNK cells were obtained from hematopoietic stem cells, and were co-cultured with or without stromal cells in the presence of IL-15 to give mNK cells (i.e., +OP9 and -OP9, respectively). The purity of stage-separated cells was determined as follows. For immunostaining of the stage-separated cells during differentiation from hematopoietic stem cells to mNK cells with antibodies directed against various cell-associated surface molecules, 1 x 10⁶ cells were counted and washed once with staining buffer solution (phosphate-buffered solution containing 20 mM HEPES, 3% fetal bovine serum, 0.1% NaN₃, pH 7.4). The cells were then reacted with NK cells differentiation-staged markers (cell markers) conjugated with FITC (fluoresceinated isothiocyanate) or PE (phycoerythrin), i.e., antibodies such as c-kit (Pharmingen), lineage (Pharmingen), NK1.1 (Pharmingen), CD122 (Pharmingen), at the temperature of 0°C for 30 minutes. After washing twice with staining buffer solution, cells were analyzed using FACS (BD/Aria). For FACS analysis, c-kit and lineage (Pharmingen) were used for hematopoietic stem cells (c-kit⁺Lin⁻), while CD122 (Pharmaingen) and NK1.1 (Pharmingen) antibodies were used for pNK cell (CD122⁺NK1.1⁻) and mNK cells (CD122⁺NK1.1⁺). The purity of hematopoietic stem cells, pNK cells, mNK cells (-OP9) and mNK cells (+OP9) was confirmed as 96%, 95%, 94%, and 96%, respectively (Fig 2).

### (C) Identification of genes specifically expressed in mouse natural killer cells

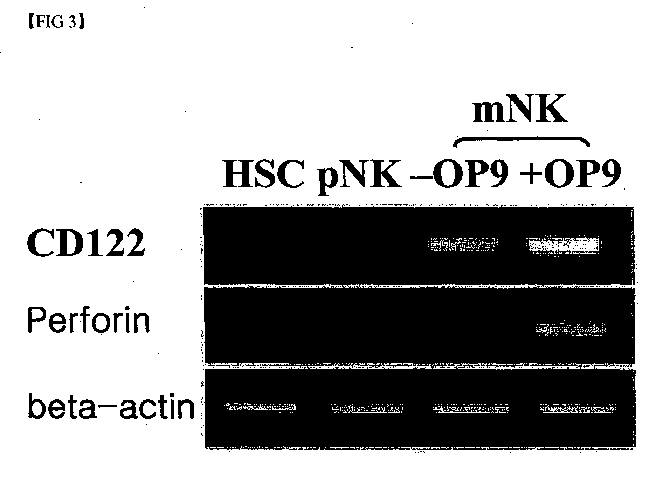

The expression of CD122 and perforin specific in natural killer cells obtained by the above step was confirmed by reverse transcriptase polymerase chain reaction (RT-PCR). The RT-PCR was performed as follows. 2 x 10⁶ LK1 cells were washed once with phosphate-buffered solution. After 500 µℓ of RNA extraction solution (RNAzol B, TEL-TEST) was added to each specimen, cells were crushed by gentle pippetting. After the addition of 50 µℓ of chloroform, the suspension was well-mixed and stood on ice for 5 minutes. It was centrifuged at 12,000 x g, 4°C for 15 minutes and the supernatant was taken and the same volume of isopropyl alcohol was added. The resulting mixture was left on ice for 20 min. Then, it was centrifuged again at 12,000 x g, 4°C for 20 minutes. The resulting deposit was washed with 80% ethanol. After dryness, it was dissolved in 20 µℓ of water containing 0.1% diethyl pyrocarbonate. From the RNA obtained thereby, single-stranded cDNAs were synthesized with MMLV reverse transcriptase (Roche). Using gene-specific primers (CD122: 5'-gtcgacgctcctctcagctgtgatggctaccata-3' and 5'-ggatcccagaagacgtctacgggcctcaaattccaa-3'; perforin : 5'-gtcacgtcgaagtacttggtg-3' and 5'-aaccagccacatagcacacat-3'; β-actin : 5'-gtggggcgccccaggcacca-3' and 5'-ctccttaatgtcacgcacgatttc-3'), RT-PCR was conducted as follows. A total 20 µℓ of a mixture consisting of 5 µℓ of 5 x reaction buffer solution (Roche), 2 µℓ of dNTP (dATP, dCTP, dGTP, TTP, each 5 µm, Roche), 1 µℓ (20 pmol) of 3 primer, 1 µℓ of distilled water, 10 µℓ of whole cellular RNA, and 1 µℓ (200 U) of reverse transcriptase was reacted at the temperature of 42°C for 30 minutes and, then, quenched at the temperature of 90°C for 5 minutes. For the PCR, the resulting solution (20 µℓ) was mixed with 8 µℓ of 10 x PCR buffer solution (Roche), 1 µℓ (20 pmol) of 5 primer, 1 µℓ (20 pmol) of 3 primer, 69 µℓ of distilled water, and 1 µℓ (2.5 U) of Taq polymerase (Takara). A total 100 µℓ of the mixture was stood at the temperature of 95°C for 5 minutes to inhibit any enzymatic activity. Using one cycle at 95°C for 90 seconds, 55°C for 60 seconds, and 72°C for 120 seconds, 30 cycles were repeated and the final reaction was performed at 95°C for 90 seconds, 55°C for 60 seconds, and 72°C for 5 minutes. 10 µℓ of aliquot was taken from the mixture obtained therefrom and was electrophoresed over 1% agarose gel at 100 V for 30 minutes. This analysis revealed that the differentiation-staged cells show the same CD122 and perforin expression pattern as previously known in the art. CD 122 was expressed more in mNK cells than pNK cells. The co-culturing with stromal cells resulted in more expression of CD 122. Perforin was expressed in mNK cells. The co-culturing with stromal cells increased the expression of the perforin (Fig 3).

### (D) Measurement of differentiation levels with or without co-culturing with mouse stromal cells

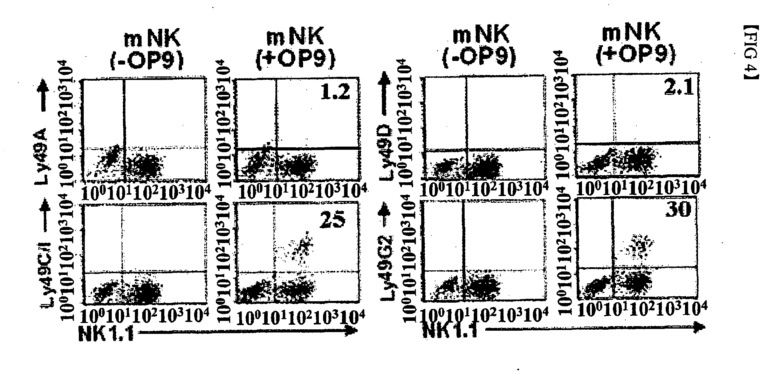

The expressions of cell-associated surface molecules (NK1.1 and Ly49) of mNK cells (-OP9 and +OP9) were compared by FACS analysis preformed in the above step (B) using NK1.1 and Ly49 antibodies (Pharmingen). As a result, mNK cells developed under co-culturing with stromal cells in the presence of IL-15 expressed more NK1.1 and Ly49 cell-associated surface molecules than those developed in the presence of IL-15 but without co-culturing with stromal cells. Therefore, stromal cells were proved to play a crucial role and present specificity in the final differentiation stage of NK cells. The mNK cells developed under co-culturing with stromal cells were composed of 1.2% Ly49A⁺NK1.1⁺, 25% Ly49C/I⁺NK1.1⁺, 2.1% Ly49D⁺NK1.1⁺, and 30% Ly49G2⁺NK1.1⁺. However, the mNK cells developed without stromal cells failed to give the cells with the same composition (Fig 4).

### (E) Identification of substances capable of inducing mouse mNK cell differentiation by SAGE

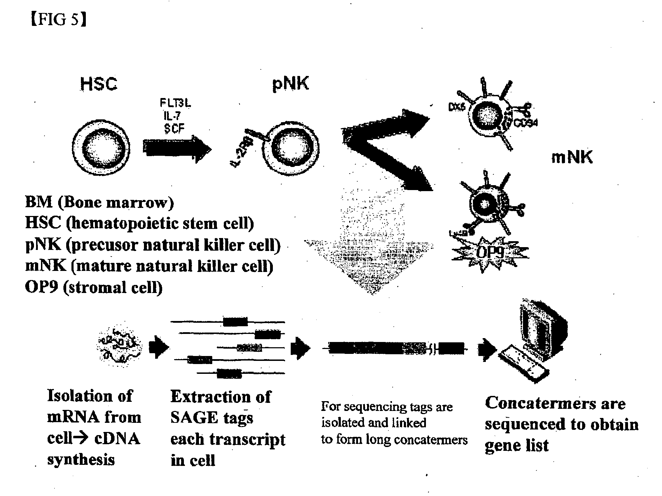

To identify the genes that are specifically expressed along each differentiation stage for the isolated HCSs, pNK cells and the two type of mNK cells, serial analysis of gene expression (SAGE) was performed (Fig 5). In accordance with the same method as the above step (C), total RNAs were extracted from the NK development stage-specific cells including HSCs, pNK cells, and two types of mNK cells. The mRNA was purified from 5 µg of total RNA with oligo (dT)₂₅ beads (Dynal A.S.) according to the manufacturer's instructions. cDNA was synthesized with a cDNA synthesis kit (Life Technologies) using 5'-biotinylated and 3'-anchored oligo (dT) primer as described in the manufacturer's protocol. SAGE tags were constructed from the cDNA and concatenated by T4 DNA ligase. The concatemers were cloned into *Sph* I-cleaved pZero-1 vector (Invitrogen) using T4 DNA ligase (Roche). After selection of positive colonies by PCR using M13 forward and M13 reverse primers, PCR products were obtained in accordance with PCR performed in the above step (C). The PCR products were sequenced with the Big-Dye sequencing kit and ABI377 sequencer (Perkin-Elmer Applied Biosystems, Branchburg, NJ). The tag sequences were extracted with SAGE 300 software. A reference SAGE-tag database was constructed from the UniGene mouse database representing most of the known mouse expressed sequences in GenBank. The conditions used for determining SAGE tags in sequences included (*i*) the orientation of each transcript, (*ii*) the presence of a poly (A) signal (AATAAA or ATTAAA), (*iii*) the presence of a poly (A) tail, and (*iv*) the presence of the CATG cleavage site in the sequence terminal. All SAGE tags extracted from the reference sequences were used for building the reference SAGE database. A computational program, GIST (Gene Identification and Sequence Topography), was developed for matching experimental SAGE tags against the reference SAGE database for identifying potential corresponding genes for each SAGE tag. This revealed that among genes expressed in pNK cells but not expressed in any other development stages, Axl gene was expressed in a great number and specifically in pNK cells (Table 1). For statistical analysis of data, p values were analyzed using a paired Student t test software program (Statview 5.1; Abacus Concepts, Berkeley, CA, USA). The statistical significance (p) in the evaluation of differential expression of SAGE tags among the samples was determined by IDEG6 analysis using the v2, Audic, and Claverie methods (http://telethon.bio.unipd.it/bioinfo/IDEG6_form).

**Table 1**

| Gene specifically expressed in pNK cells | | | | | |
|---|---|---|---|---|---|
| Gene name | HSC | pNK | mNK(-OP9) | mNK(+OP9) | Unigene Id |
| | 0 | 189 | 0 | 0 | Mm.4128 |

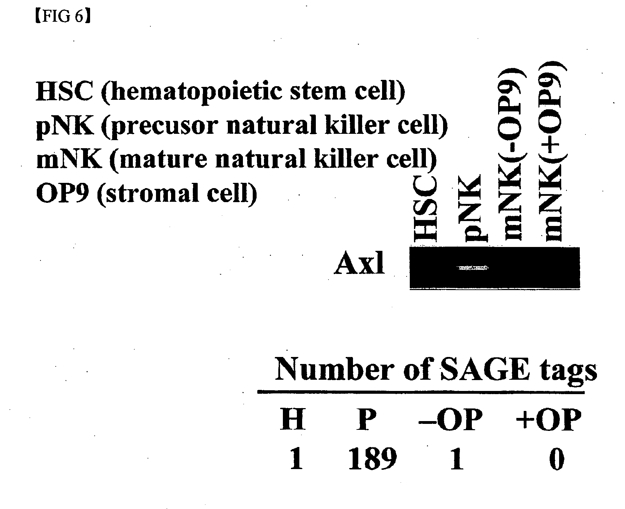

The RT-PCR was conducted to confirm whether the Axl gene expression in pNK cells is substantially consistent with the result of SAGE. As a result, it is found that the number of SAGE tag was consistent with the expression pattern of Axl. This shows that the amount of the Axl expression was augmented in pNK cells (Fig 6).

### Example 2

### Effect of Axl polyclonal antibody on the differentiation of natural killer cell

### (A) Expression of mature natural killer cell-associated receptors by Axl polyclonal antibody: Co-culturing with stromal cells

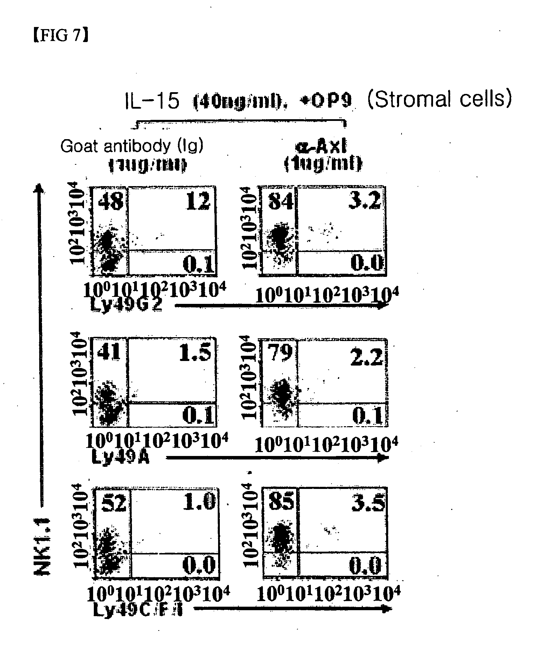

During the differentiation from mouse hematopoietic stem cells to natural killer cells, precursor natural killer cells (on day 7) were treated with 1 µg of Axl polyclonal antibody (Santa Cruz Biotechnology, Inc. sc-1096) and, in accordance with the same method as described above, the pNK were co-cultured with stromal cells in the presence of IL-15 (40 ng/mℓ) for 6 days. The developed natural killer cells were stained with NK1.1 antibody and antibodies directed against natural killer cell-associated receptors (Ly49G2, Ly49A, Ly49C/F/I, Pharmingen) and were analyzed by FACS in accordance with the same method as described in Example 1(B). The test cells treated with Axl polyclonal antibody were compared with control cells treated with goat Ig (R&D) and none-treated (obtained by the method of Example 1(B)). As a result, it was found that, the level of differentiation was increased from 1.2% to 3.2% in Ly49A⁺NK1.1⁺ cell, 1% to 3.5% in Ly49C/I⁺NK1.1⁺ cell, and 1.2% to 3.2% in Ly49G⁺NK1.1⁺ cell, respectively. This proves that Axl polyclonal antibody augmented the development of mature natural killer cells by about two times and it positively affects the differentiation from pNK cells to mNK cells (Fig 7).

### (B) Expression of mature natural killer cell-associated genes by Axl polyclonal antibody: Co-culturing with stromal cells

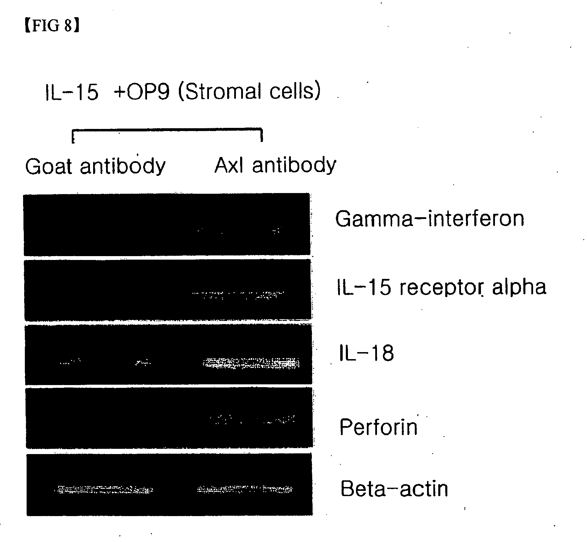

The expression of mature natural killer cell-associated genes (interferon-γ, 5'-agcggctgactgaactcagattg-3' and 5'-gcacagttttcagctctatagg-3' IL-15Ra, 5'-ccaacatggcctcgccgcagct- 3' and 5'-ttgtagagaaagcttctggctc-3' IL-18, 5'-aggtacaaccgcagtaatgcgg- 3' and 5'-agtgaacattacagatttatccc-3' and perforin, 5'-gtcacgtcgaagtacttggtg-3' and 5'-aaccagccacatagcacacat-3' in the same natural killer cells as described in the above (A) was analyzed by the PCR as described in the above Example 1(C). As a result, the test cells treated with Axl polyclonal antibody shows an increase in the expression of the above genes compared to the control treated with goat antibody. This demonstrates that the Axl polyclonal antibody positively affects the differentiation into completely mature natural killer cells (Fig 8).

### (C) Expression of mature natural killer cell-associated receptors by Axl polyclonal antibody: Culturing without stromal cells

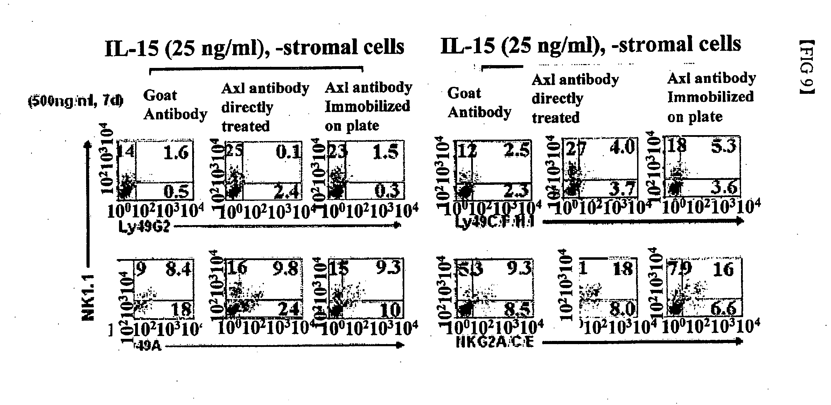

In order to confirm the effect of Axl polyclonal antibody on the development of mature natural killer cells under the different condition, pNK cells were cultured with the treatment of lower concentration of 25 ng/mℓ of IL-15 and 500 ng/mℓ of Axl polyclonal antibody but without co-culturing with stromal cells, while being fixed onto the culture dish. The developed cells were stained with NK1.1 antibody and antibodies directed against natural killer cell-associated receptors (Ly49G2, Ly49A, Ly49C/F/I, NKG2A/C/E) and were analyzed by FACS in accordance with the same method as described in Example 1(B). As results, compared to the cells not treated with Axl polycolnal antibody, it is found in the cells treated with the antibody that the differentiation increased from 8.44% to 9.8% and 9.3% in Ly49A⁺NK1.1⁺ cell, from 2.5% to 4% and 5.3% in Ly49C/F/I⁺NK1.1⁺ cell, and from 9.3% to 18% and 16% in NKG2A/C/E+NK1.1⁺ cell, respectively (Fig 9).

### (D) Expression of mature natural killer cell-associated genes by Axl polyclonal antibody: Culturing without stromal cells

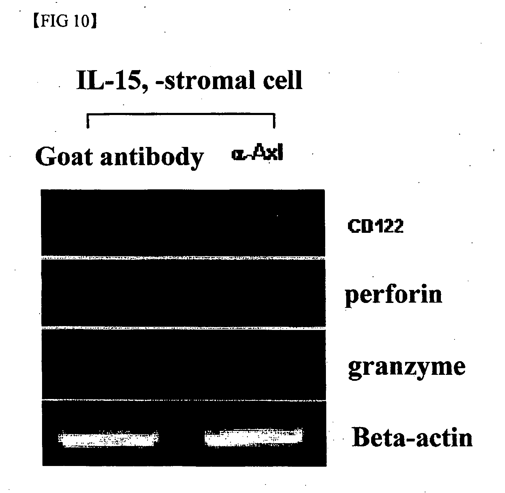

The expression of mature natural killer cell-associated genes (CD 122, perforin, and granzyme B) in the same natural killer cells as described in the above (A) was analyzed by the RT-PCR as described in the above Example 1(C). As a result, the test cells treated with Axl polyclonal antibody increased the expression of the above genes compared to the control. This demonstrates that the Axl polyclonal antibody positively affects the differentiation into completely mature natural killer cells even in the absence of stromal cells (Fig 10).

### Example 3

### Effect of Axl polyclonal antibody on interferon-γ production by natural killer cells

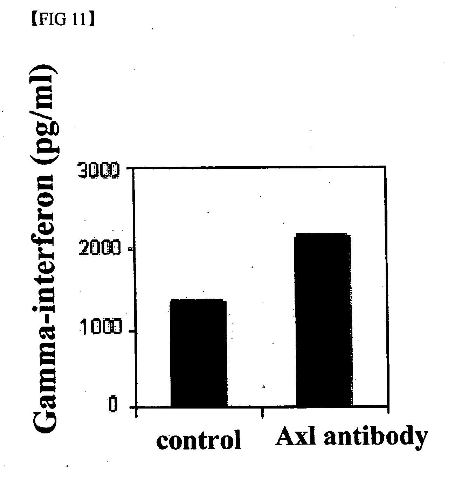

The pNK cells obtained in the above Example 1(B) were co-cultured with stromal cells in the presence of Axl polyclonal antibody (500 ng/mℓ) and IL-15 (10 ng/ mℓ) to produce mature natural killer cells, which were then activated with the treatment of IL-2 (10 ng/mℓ, R&D). After 24 hours, the amount of the produced interferon-γ was measured using interferon-γ ELISA kit (BD Pharmingen). As a result, it was shown that the test cells treated with Axl polyclonal antibody increased the amount of the produced interferon-γ compared to the control (Fig 11).

### Example 4

### Effect of Axl polyclonal antibody on the proliferation of pNK cells

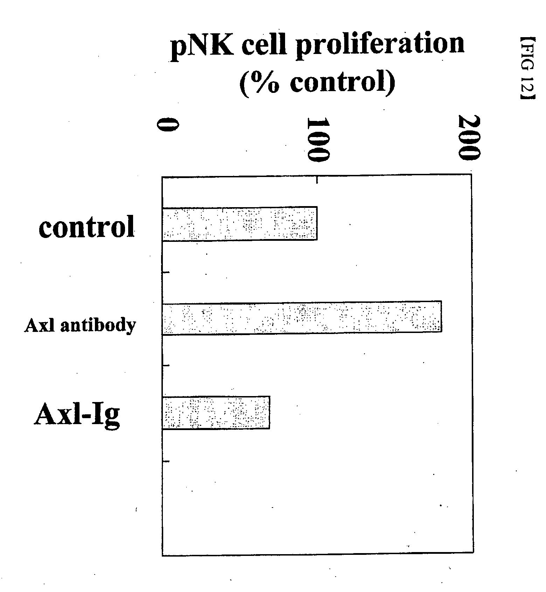

Stromal cells were plated in 96-well microtiter plate. After one day, pNK cells obtained in the above Example 1(B) were seeded to 2.5 x 10⁴cells/well and were treated with IL-15 (25 ng/mℓ) along with 500 ng/mℓ of goat antibody, Axl polyclonal antibody (α -Axl) or Axl-Ig. After 48 hours, the proliferation of pNK cells was analyzed by MTS assay (CellTiter 96 Aqueous Assay, Promega, Madison, WI). The MTS is a colorimetric assay using tetrazolium compound [3-(4,5-dimethylthiazol-2-yl)-5-(3-carboxymethoxyphenyl)-2-(4-sulfophenyl)-2H-tetrazolium, inner salt; MTS] and electron-coupling reagent phenazine methosulfate (PMS). On the last day of cell culture, 0.025 mℓ of MTS/PMS mixture solution was added to each well and was reacted for 30 minutes. Then, optical absorbance was measured at the wavelength of 495 nm using ELISA Reader (Molecular Devices Co., Sunnyvale, CA, USA). It was found that the proliferation of pNK cells treated with Axl polyclonal antibody is about twice as much as the control. In the meantime, the proliferation of pNK cells treated with Axl-Ig was reduced. This demonstrates that the transfer of Axl signal by Axl polyclonal antibody has a significant effect on the proliferation of pNK cells (Fig 12).

### Example 5

### Effect of the interaction of Axl and its ligand Gas6 on the differentiation of natural killer cells

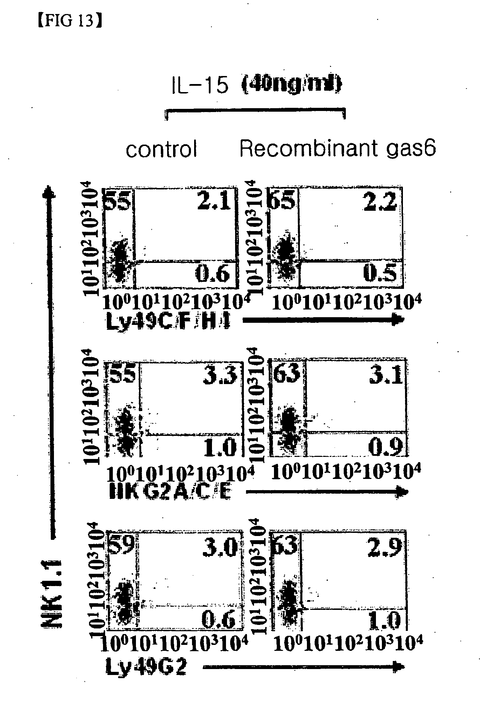

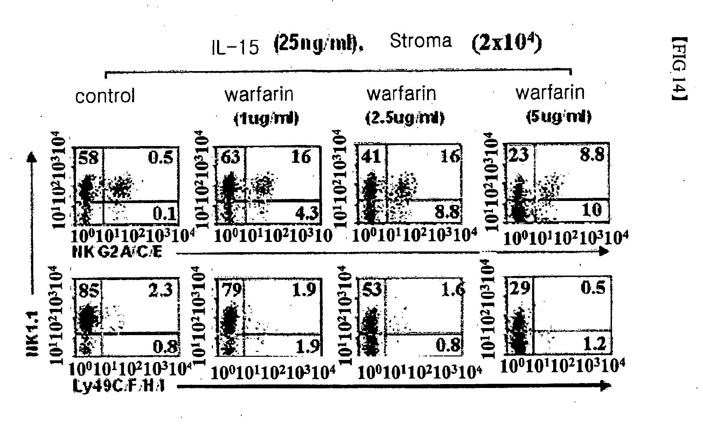

In order to elicit the effect of the signal transfer by the binding between Gas6 and Axl on the differentiation of natural killer cells, mouse recombinant Gas6 (500 ng/mℓ, R&D) was added during the differentiation. However, it did not affect the differentiation of natural killer cells (Fig 13). It was understood that Gas6 needs to be γ-carboxylated for exhibiting its biological activity. For next experiments, Gas6 expressed in stromal cells was used. The effect of Gas6 on the signal transfer through Axl was examined by using warfarin (Sigma) to inhibit the action of Gas6 as the ligand of Axl expressed in pNK cells. The pNK cells differentiated from HSCs were treated with IL-15 (25 ng/mℓ) along with 1 µg/mℓ, 2.5 µg/mℓ, and 5 µg/mℓ of warfarin and were co-cultured with stromal cells (2 x 10⁴) for 7 days. The level of differentiation from pNK cells to mNK cells was analyzed by FACS as described in the above Example 1(B). As a result, the receptors specifically expressed in NK1.1, NKG2A/C/E and Ly49C/F/H/I natural killer cells were decreased in a concentration-dependent manner (25%, 16%, 16%, and 8.8% for NK1.1⁺NKG2A/C/E⁺; and 2.3%, 1.9%, 1.6%, and 0.5% for NK1.1⁺Ly49C/F/H/I⁺) (Fig 14). This proves that the interaction between Axl and its ligand Gas6 plays a crucial role in the differentiation from pNK cells to mNK cells.

### Example 6

### Construction of mouse Gas6 expression vector and retrovirus vector

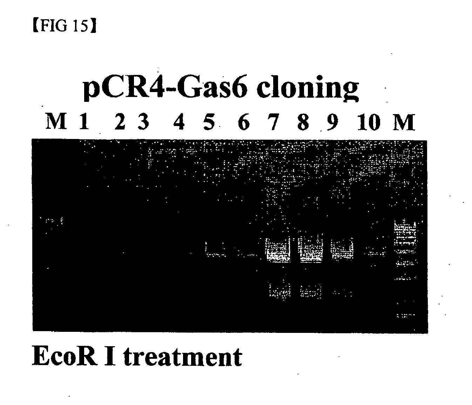

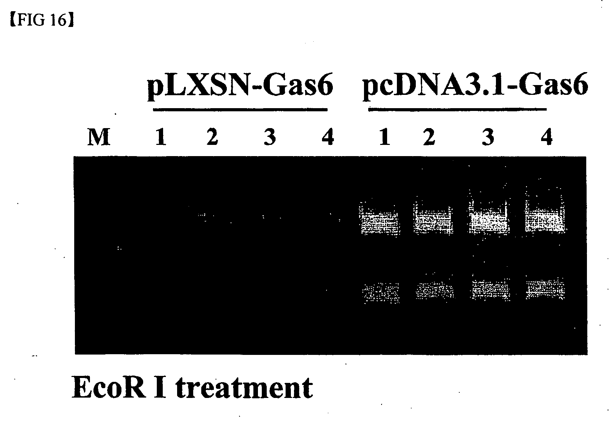

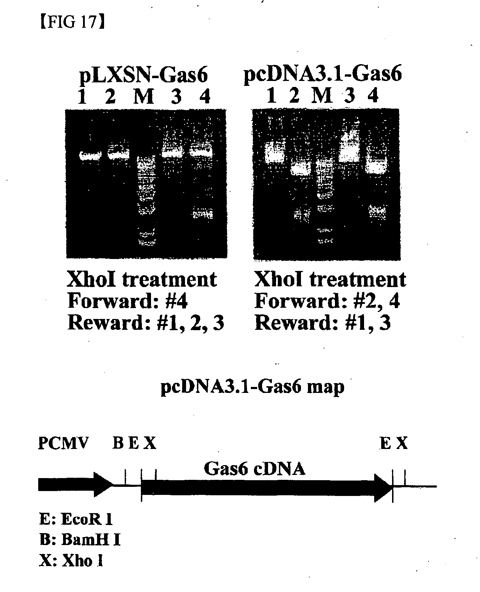

To produce active Gas6, RT-PCR was performed using the RNAs extracted from mouse stromal cells and Gas6 primers ( 5'-ggcctcgagcatgccgccaccgcccgggc -3'and 5'-ggcgaattccggtctagggggtggcatgc-3' as described in the above 1(C) to amplify Gas6 cDNA (100 ng), which was cloned into pCR4-TOPO (50 ng, Invitrogen) digested with restriction enzyme EcoRI (1U, Roche) (Fig 15). After the clones were sequenced, the cDNA of the same clone as reference sequence (gene ID; AK086187) was used to construct mouse Gas6 expression vector and retrovirus vector. Retrovirus vector pLXSN (Invitrogen) was digested with EcoRI (1U). To this was added mouse Gas6 cDNA (100 ng) which had been isolated by digesting pCR4-Gas6 #8 with EcoRI (1U). Then, T4 DNA ligase was treated to construct a recombinant pLXSN-Gas6 (Fig 16, left). pcDNA3.1(+) was digested with EcoRI(1U) and CIP(calf intestinal alkaline phosphatase). To this was added mouse Gas6 cDNA(100 ng) which had been obtained by treating pCR4-Gas6 #8 with EcoRI(1U). The mixture was reacted with T4 DNA ligase (1U) to construct a recombinant pcDNA3.1-Gas6 (Fig 16, Right). In these constructed expression vectors, the direction of mouse Gas6 cDNA to virus and CMV promoter was confirmed by using Xhol (1U, Roche). The clones in which the cDNA was forwardly and reversely positioned to the promoter were designated as pLXSN-Gas6/F, pLXSN-Gas6/R and pcDNA3.1-Gas6/F, pcDNA3.1-Gas6/R, respectively (Fig 17).

### Example 7

### Preparation of mouse Gas6 transfectant

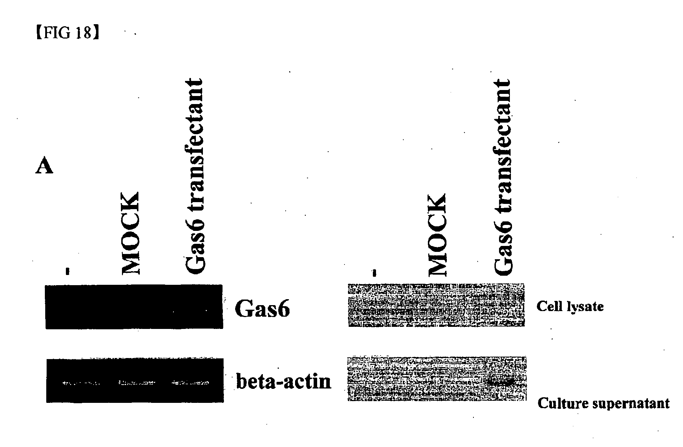

NIH3T3 cell line (ATCC) which does not express Gas6 gene was transfected with pcDNA3.1(+), pcDNA3.1-Gas6/F and pcDNA3.1-Gas6/R using lipofectamine (Invitrogen). The transfectants were screened on the medium containing antibiotics G418 and were limitedly diluted to give the clones having the mouse Gas6 gene overexpressed. In the meantime, for the construction of mouse Gas6-expressing retrovirus, Gas6 retrovirus vectors pLXSN-Gas6/F and pLXSN-Gas6/R were transfected into PT67 cell line. The transfectants were screened on antibiotics G418-containing medium and were limitedly diluted to give retrovirus-infected NIH3T3 cell line. The expression level of Gas6 was confirmed by performing the same RT-PCR (A) as described in the above Example 1(C) using Gas6 primers (5'-ggcctcgagcatgccgccaccgcccgggc-3' and 5'-ggcgaattccggtctagggggtggcatgc-3' and Western blott analysis (B) (Fig 18). The Western blot analysis was carried out as follows. The cells were collected from a culture dish and were treated with lysis buffer (20 mM HEPES pH 7.9, 100 mM KCl, 300 mM NaCl, 10 mM EDTA, 0.5% Nonidet P-40, 1 mM Na₃VO₄, 1 mM PMSF, 100 mg/mℓ aprotinin and 1 mg/mℓ leupeptin). The lysate was stood on ice for 30 minutes. The protein concentration was measured using Bradford reagent (Bio-Rad, Hercules, CA). An equivalent amount of protein lysate was electrophoresed over 10% SDA-PAGE and then the electrophoresed proteins were transferred to PVDF membrane (Millipore, Marlborough, MA). The PVDF membrane was reacted with a blocking buffer solution (1% BSA and 5% skim milk in PBS) at the temperature of 4°C overnight, and then washed three times with TBST (50 mM Tris. pH 7.4, 150 mM NaCl, 0.05% Tween 20). The PVDF membrane containing the protein was treated first with goat-anti-mouse Gas6 antibody (Santa Cruz) at room temperature for 1 hr and then treated second with HRP conjugated anti-goat IgG (Santa Cruz) at room temperature for another 1 hr. After washing three times the membrane with TBS, signal detection was carried out using ECL system (Amersham-Pharmacia Biotech, Arlington Height, IL).

### Example 8

### Effect of murine Gas6 on the differentiation of natural killer cells

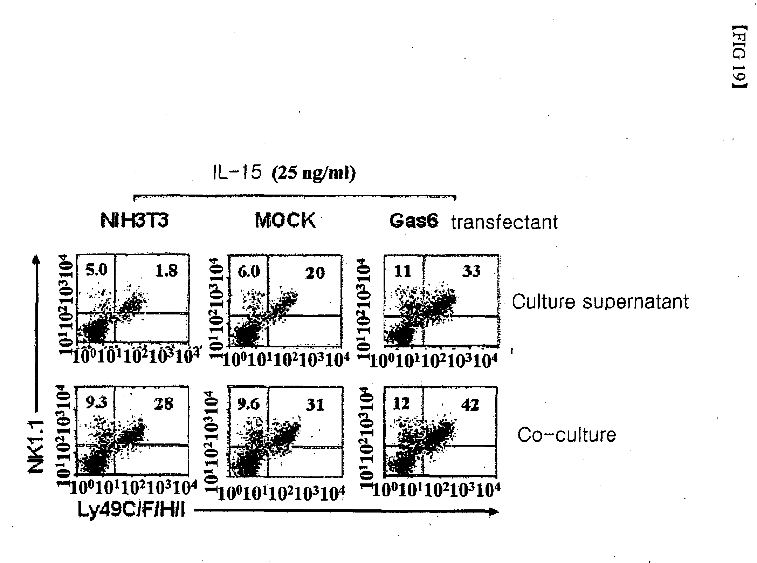

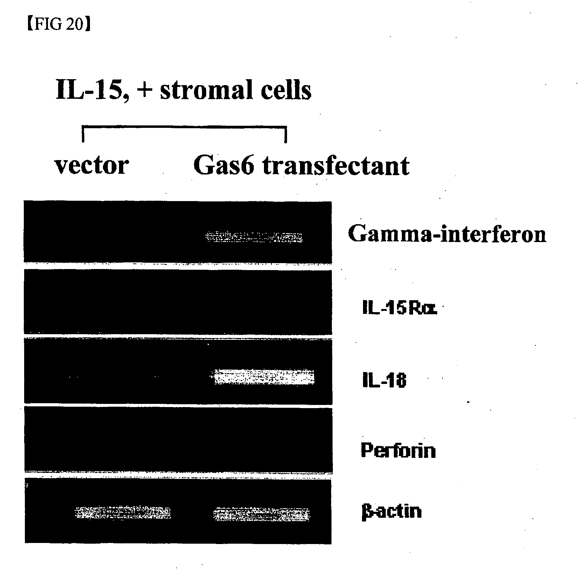

HCS cells, that are Lin- and c-kit⁺ and isolated from mouse bone marrow, were cultured in 24-well plate at the concentration of 1 x 10⁶/mℓ with RPMI medium containing SCF (30 ng/mℓ), IL-7 (0.5 ng/mℓ), Flt31 (50 ng/mℓ), indometacine (2 µg/mℓ), and gentamicin (2 µg/mℓ). After culturing for 6 days under the condition of 37°C, 5% CO₂, the resulting pNK cells were cultured with IL-15 (20ng/ml) together with murine Gas6 transfectant for 24 hrs. The resulting mixture was centrifuged for 10 min at 2000rpm and the supernatant culture sample was again cultured for another 6 days with murine Gas 6 transfectant and Axl polyclonal antibody for the differentiation into mNK cells. The resulting cells were harvested and tested for the expression of mNK surface molecules with the same method used in Examples 1(B). Purity of the cells at each stage was all at least 95% based on FACS analysis. In order to confirm whether murine Gas6 protein secreted from the murine Gas6 transfectant affects the differentiation into mNK cells in combination with Axl protein, the degree of differentiation of NK cells was determined for the control group in which pNK cells were transfected with normal NIH3T3 cells and the vector and the group co-cultured with the transfectant which over-expresses murine Gas6 protein or with the culture supernatant of said transfectant. As a result, it was found that, pNK cells co-cultured with murine Gas6 transfectant have greater differentiation compared to the control group (i.e., increased from about 14% to 47%, see Fig 19). In addition, when the culture supernatant comprising the transfectant (5x10⁶) which over-expresses Gas6 protein compared to the control group was added during the process of differentiation (diluted 1:20 ratio), perforin, IL-18 and interferon-γ specifically expressed in mNK cells were expressed more, according to the analysis the same as described in Example 1(C) (Fig 20). Therefore, it is confirmed that in conjunction with Gas6 protein Axl exerts a significant effect on the differentiation of HCS cells into NK cells via signal transfer.

### Example 9

### Effect of Gas6 on the production of interferon-γ by NK cells

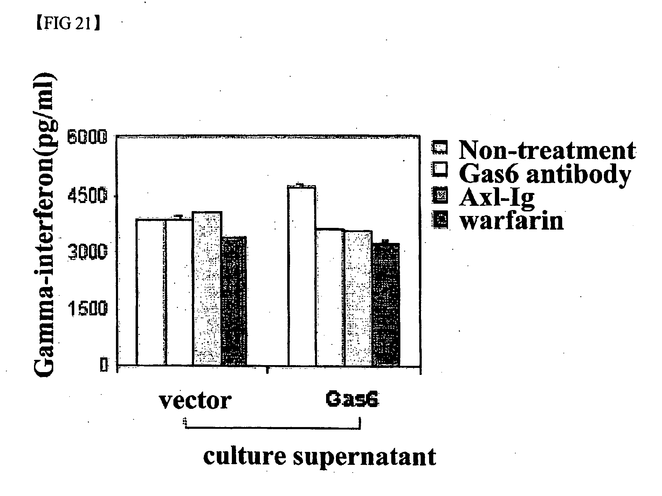

pNK cells obtained from Example 1(B) was co-cultured with IL-15 (10 ng/mℓ) and stromal cells. Gas6 (500 ng/mℓ), Axl-Ig (500ng/ml) and warfarin (500ng/ml) were added. After obtaining mNK cells respectively from the test group in which 1:20 dilution of the supernatant of Gas6 transfectant was treated and from the control group in which 1:20 dilution of the supernatant of vector-only-transfectant was treated, the cells were treated with human IL-2 (10ng/ml, R&D). After 24 hrs, the amount of interferon-γ produced was determined with the interferon-γ ELISA kit (BD Pharmingen). As a result, it was found that there is an increase in interferon-γ produced in the test group in which 1:20 dilution of the supernatant of Gas6 transfectant was added. Compared to the non-treated group, it is found for the test group treated with Gas6 (500 ng/mℓ), Axl-Ig (500ng/ml) and warfarin (500ng/ml) that the amount of interferon-γ produced is decreased. Thus, it is confirmed that Gas6 signal is transferred by Axl protein and therefore becomes involved in the activation of NK cells (Fig 21).

### Example 10

### Effect of Gas6 on the proliferation of pNK cells

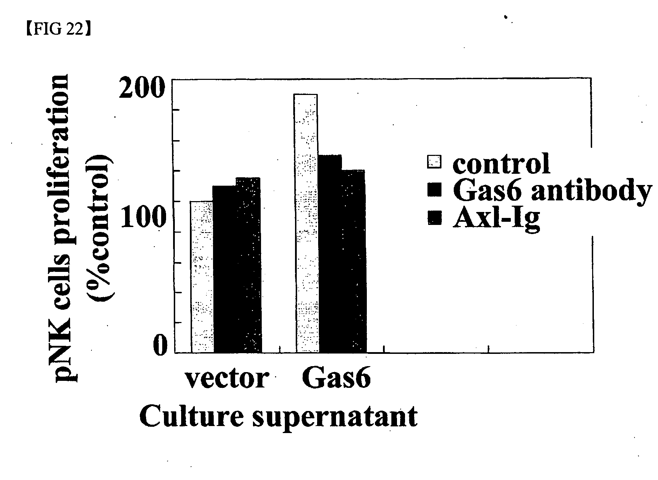

Stromal cells were plated in 96-well microtiter plate. After one day, pNK cells obtained in the above Example 1(B) were seeded to 2.5 x 10⁴cells/well. Under the condition of comprising IL-15 (25 ng/mℓ) in all culture plates, the cells were treated with 500ng/ml of Axl-Ig and Gas6 antibody (a-Gas6), respectively, and the supernatant of Gas6 culture obtained from Example 8 was added. After 48 hrs, the proliferation of pNK cells was analyzed by MTS assay, the same as described in Example 4. Compared to the control group treated with the supernatant obtained from the culture transfected with the vector only, the test group treated with the supernatant of the transfected culture in which Gas6 protein was over-expressed exhibits an increase in pNK proliferation. Further, considering that the proliferation of pNK cells is reduced by Axl-Ig and an antibody directed against Gas6, it is confirmed that the proliferation of pNK cells is induced by the interaction between Axl and Gas proteins (Fig 22).

### Example 11

### Construction of murine Axl expression system

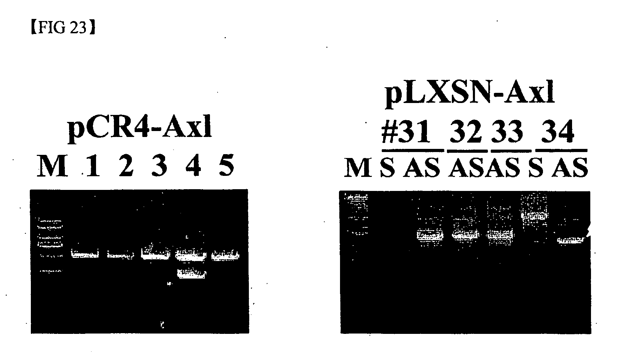

Total RNA isolated from murine RAW264.7 macrophage which highly expresses Axl protein and Axl primer (sense; 5'-ggtgcccatcaacttcggaa, antisense; 5'-ggatgtcccaggtggaagatt) were used to carry our the RT-PCR reaction the same as described in Examples 1(C). 2,750 bp murine Axl cDNA(100ng) was cloned into pCR4-TOPO(50ng) which has been digested with EcoRI(1U) (Fig 23). After digesting retrovirus vector pLXSN with the restriction enzymes of EcoRI(1U) and CIP(1U), Axl cDNA(100ng) obtained from the treatment of pCR4-Axl #4 with EcoRI restriction enzyme was added and T4 DNA ligase (1U) was added. Recombinant pLXSN-Axl was thus obtained. The direction of Gas6 cDNA relative to the promoter in the retrovirus vector was confirmed by PCR reaction using the primer downstream of the-cloning site and the Axl primer and by base sequencing method. The clones in which the cDNA was forwardly and reversely positioned to the promoter were designated as pLXSN-Axl/F and pLXSN-Axl/R, respectively.

### Example 12

### Production of murine Axl-IgG fusion protein

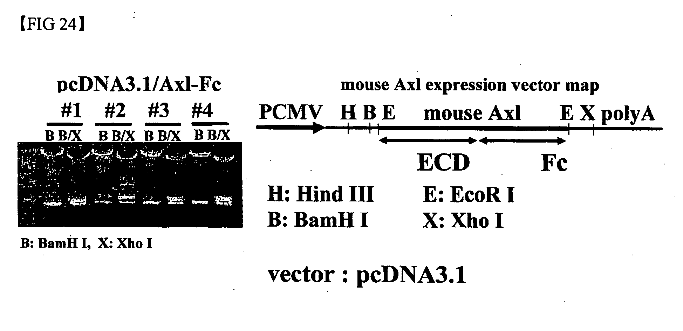

Transfection of PT67 cell line was carried out with the same method described for the production of the retrovirus expressing Gas6 protein. In order to prepare cell line expressing Axl-IgG fusion protein, at the 5' and 3'ends of murine IgGI Fc (constant fragment) BamHI and XhoI linkers were introduced respectively, the-PCR was carried out the same as Example 1(C) for the amplification, and cloned into pCR4-TOPO (pCR4-Fc). Meantime, at the 3'end of extracellular domain (ECD) of Axl gene BamHI linker was introduced and amplified, and then cloned into pCR4-TOPO (pCR4-Axl/ECD). pCR4-Fc was treated with Xho I(1U) and BamHI(1U, Roche) to separate the Fc fragment of 820bp. pCR4-Axl/ECD was treated with EcoRI(1U) and BamHI(1U), and the resulting 1350 bp Axl/ ECD(100ng) and the above obtained Fc fragment were cloned into the EcoRI-XhoI site of pcDNA3.1(50ng). DNA clones were then prepared using the restriction enzymes of BamHI(1U), BamHI(1U)/XhoI(1U) (Fig 24). Finally, 293T cells were transfected with plasmid pcDNA3.1/Axl-Fc, the transfectants were selected from G418 containing culture media, and the clones which over-express Axl-IgG fusion protein was obtained by limiting dilution method.

### Example 13

### Effect of murine Axl-IgG fusion protein on the differentiation ofNK cells

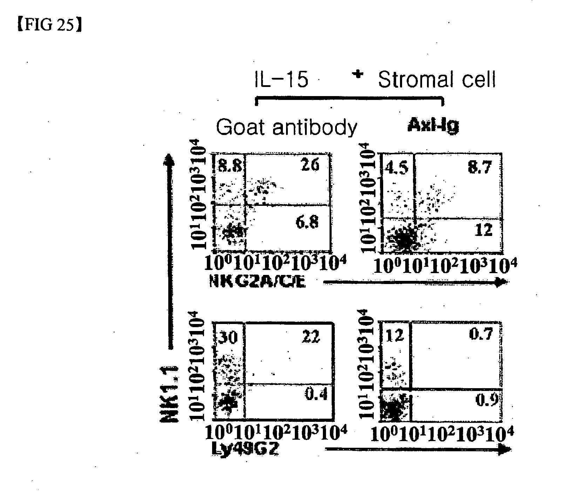

In order to determine the effect of blocking the signal transfer by Axl on the development of natural killer cells, IL-15 (25ng/ml) and Axl-Ig were treated while the differentiated pNK cells were being co-cultured with stromal cells (2x10⁴) for seven days. Subsequently, the level of differentiation of the pNK cells into mNK cells was determined by FACS analysis following staining with antibodies of NK1.1, NKG2A/C/E, and Ly49G2. As a result it was observed that, when the action of Axl and Gas6 which is a ligand for Axl protein was blocked by the addition of Axl-Ig, the number of cells positive for NK1.1, NKG2A/C/E and Ly49G2, which are markers of mNK cells, was reduced. Thus, it is found that Axl-Gas6 interaction plays an important role in the differentiation of pNK cells into mNK cells (Fig 25).

### Example 14

### Differentiation of NK cells by inhibiting Axl protein expression

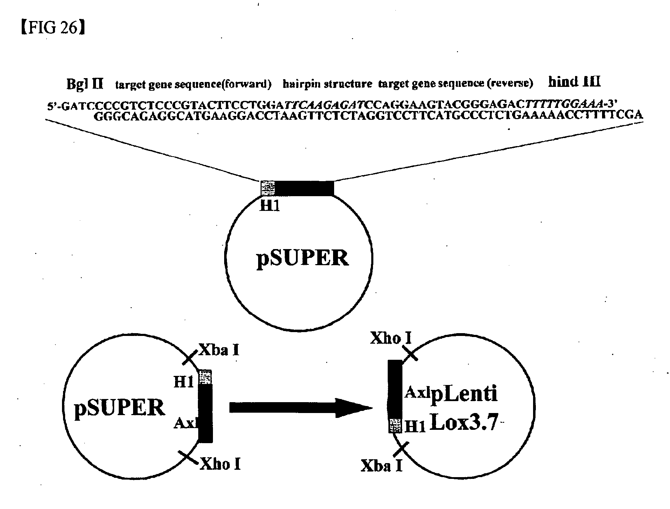

Lenti virus vector was used to remove Axl from NK cells (Fig 26).

### (1) Preparation of siRNA

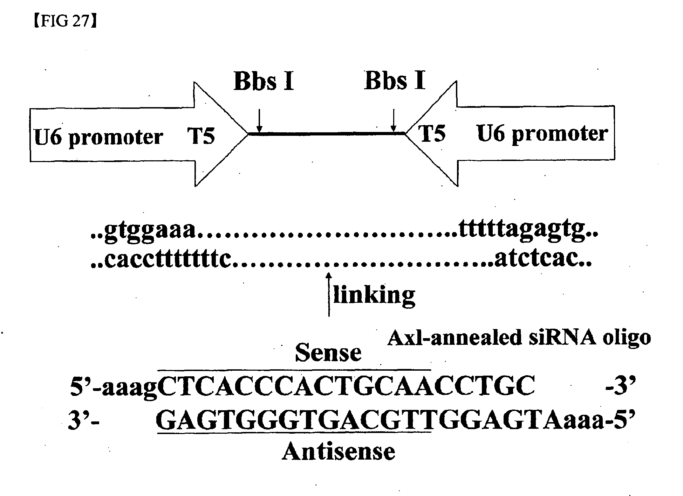

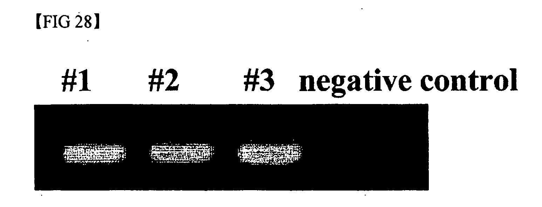

To selectively block the site with Axl activity, an oligonucleotide which can remove the function of Axl gene was prepared as follows. A target base sequence for blocking was set as gtctcccgtacttcctgga(#1), ctcacccactg caacctgc(#2), agacctacacagtttcctc(#3). For said three types of base sequence, one primer which comprises bases of aaag overhanging at 5 end in a sense direction and the other primer which comprises bases of aaaa overhanging at 3 end in an antisense direction were prepared, respectively. Each of these oligomers were annealed at 95 °C to prepare a double strand, while double promoter pFIV-H1/U6 siRNA-GFP was digested with BbsI(Roche), and then purified and cloned (Fig 27). Among the clones obtained after the transfection, PCR was carried out using U6 PCR primer and anti-sense siRNA olilgonucleotide in order to find out the clones containing siRNA sequence. As a result, for the clones containing siRNA sequence, PCR product of about 100bp was confirmed (Fig 28).

### (2) Establishment of Lenti virus siRNA expression system

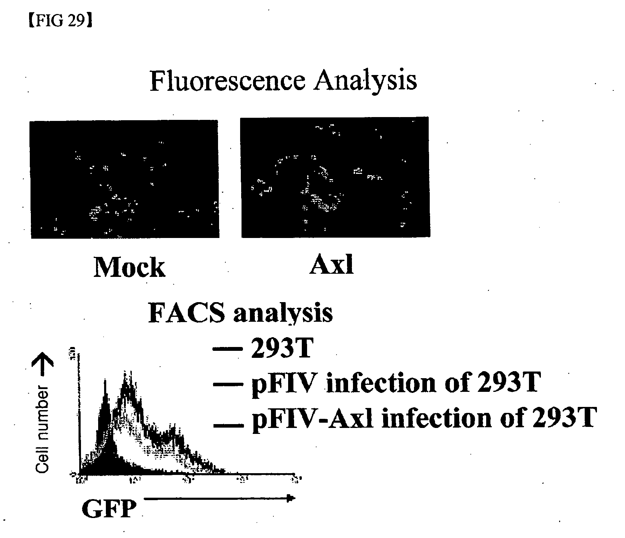

For preparing Lenti virus which can transfect NK cells, Lenti virus vector expressing siRNA and helper vectors of VSVG, RSV-REV, and pMDL g/pRRE were used. 292T cells (ATCC) which can form virus constructs were added to 10² cm cell culture dish in an amount of about 5x10⁵ cells. After about 24 hrs, the cells were transfected with the vectors prepared above, each with 1.5 µg of lipofectamine. New DMEM culture medium was supplied about 4 hrs later, and culturing was continued in CO₂ incubator at 37°C. About 48 hrs later, the culture media was centrifuged for 5 min at 3000 rpm and the cellular debris was removed. Culture supernatant was filtered through Millex-HV 0.45 µm PVDF filter system and stored. For the confirmation of the virus production from thus obtained culture supernatant, 293T cells were seeded to 2.5 x 10⁴cells/well of 6-well culture dish. After 24 hrs of culturing, virus particles were mixed with DMEM media containing 10% fetal bovine serum in 1:1 ratio and were treated into 293T cells. At this moment, in order to increase the degree of binding of virus capsid to the cellular membrane, 8 µg/ml of polybrene was further added. After the trensfection with the virus for 24 hrs, the media was replaced with new DMEM media containing 10% fetal bovine serum. After another 48hrs of culturing, fluorescence spectroscopy and FACS analysis as in Example 1(B) were carried out. As a result, it was confirmed that the cells were successfully transduced with pFIV expression vector tagged with GFP (Fig 29).

### (3) Transfection with Lenti virus during the differentiation of hematopoietic cells into natural killer cells

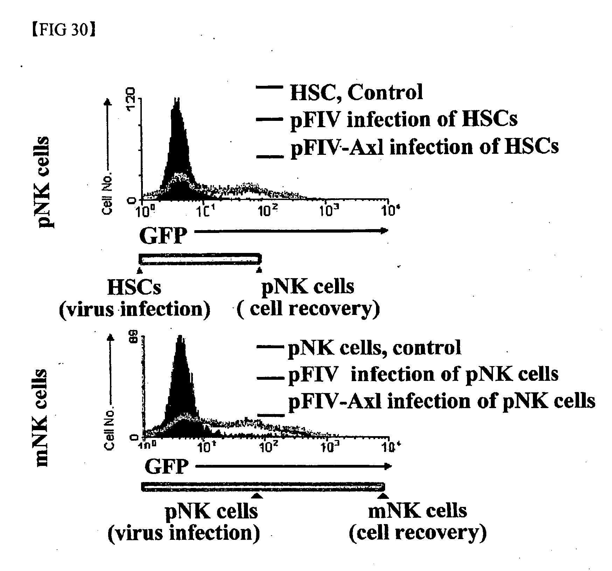

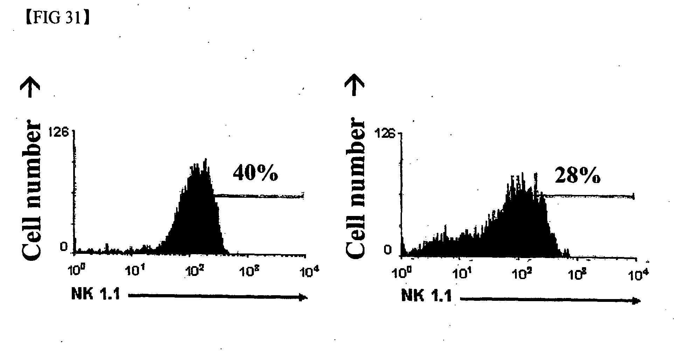

Hematopoietic cells were isolated from mouse bone marrow. Virus particles were added to the cells in an amount of about 1x10⁶ particles per cell and the transfection was carried out for 24 hrs. After culturing the cells in the media comprising IL-7 (0.5ng/ml), FLT3L (50ng/ml) and SCF(30ng/ml) for about 6 days, precursor NK cells were obtained. According to the FACS analysis as described in Example 1(B), GFP expression was increased thanks to good transfection by Lenti virus and at the same time, the differentiation of NK cells was inhibited by Axl inhibition (Fig 30 and Fig 31).

### Example 15

### Determination.of inhibitory activity of NK cells on tumorigenesis in a cancerous animal model

For establishing a cancerous animal model, B16F10 melanoma cells which are cancer cells originating from C57BL/6 were injected intravenously to 7 to 8 week old C57BL/6 mice (5x10⁴ cells per animal). Next day, mNK cells differentiated by human IL-2(10ng/ml, R&D) and a control antibody (1 µg/ml), or by IL-2(10ng/ml) and Axl polyclonal antibody (1 µg/ml), respectively, were intravenously injected into the animal (1x10⁶ cells per animal). Two weeks later, migration of the cancer cells and anticancer efficacy were determined by measuring the number of B16F10 melanoma cells found in the animal's lung. As a result, number of cancer colonies found in the lung tissues injected with mNK cells that have been stimulated by Axl polyclonal antibody was reduced compared to that of the mice injected with mNK cells treated with the control antibody (i.e., 4 to 10 fold decrease). However, for the lung tissues injected with mNK cells treated with Axl-Ig, number of cancer colonies were significantly increased, confirming that Axl can regulate not only the formation of cancer cells by activating NK cells but also the migration of the cancer cells.

### Example 16

### Determination of tumoricidal activity of NK cells in a cancerous animal model

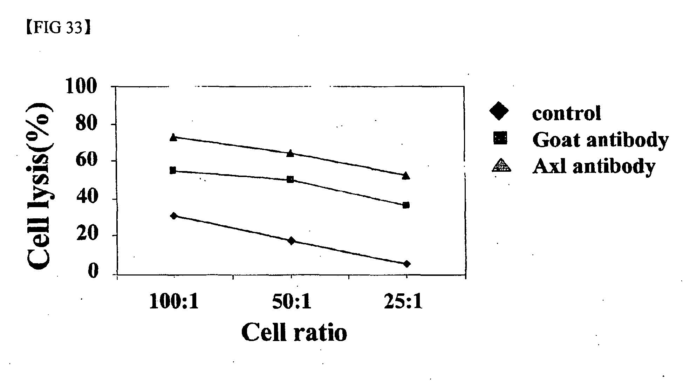

In order to compare tumoricidal activity of NK cells, spleen cells were separated from the mouse of Example 15, stimulated with human IL-2 (10ng/ml) for 48 hrs, and treated into YCA-1 cells tagged with ⁵¹Cr (100µci) for 2 hrs in ratio of 1:100, 1:50 and 1:25. After 4 hrs of incubation, the amount of ⁵¹Cr released to the culture supernatant was measured. As a result, tumoricidal activity was found for each group as follows; for the control group it was about 30%, for the test group in which the NK cells differentiated by treating with control antibody were injected it was 58%, and for the test group in which the NK cells differentiated by treating with Axl polyclonal antibody were injected it was about 75%, which is significantly higher than that of the control group (Fig 33).

### Example 17

### Survival rate analysis of cancer-induced mouse after the treatment with NK cells in a cancerous animal model

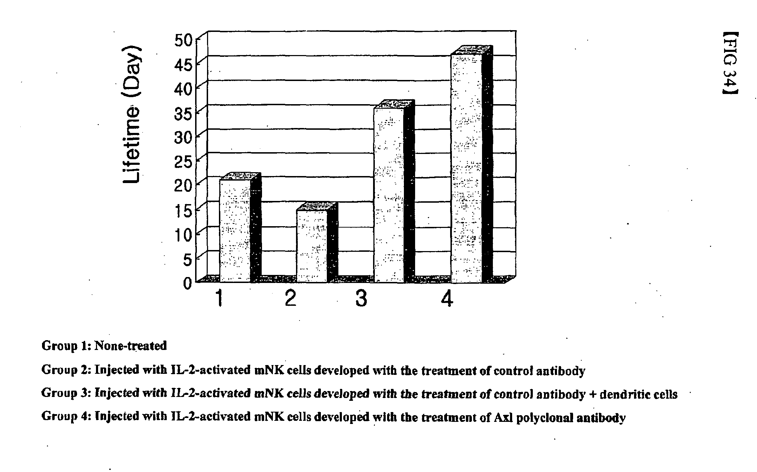

By subcutaneously injecting B16F10 melanoma cells which are cancer cells originating from C57BL/6 in an amount of 5x10⁴ cells per animal, cancer was induced in 7 to 8 week old C57BL/6 mice. For the first group of mice, no further substance was injected. For the second group, mNK cells differentiated by the treatment with a control antibody (1 µg/ml), and human IL-2(10ng/ml, R&D) were further subcutaneously injected. For the third group, mNK cells differentiated by the treatment with a control antibody (1 µg/ml) and human IL-2(10ng/ml, R&D) and dendritic cell were further subcutaneously injected. For the fourth group, mNK cells differentiated by the treatment with Axl polyclonal antibody (1 µg/ml) and human IL-2(10ng/ml, R&D) were further subcutaneously injected. The amount of the injected mNK cells was 1x10⁶ per animal. As a result, it was found that survival days of the mice were 21, 15, 36 and 47, respectively, for said four groups in order. Thus the mice treated with mNK cells differentiated by the treatment with Axl polyclonal antibody (1 µg/ml) and human IL-2(10ng/ml, R&D) survived the longest, and considering it is longer than that of the mice injected together with the dendritic cells, it is believed that differentiated mNK cells are more effective for cancer treatment (Fig 34).

### Example 18

### Construction of human Axl E.coli expression vector

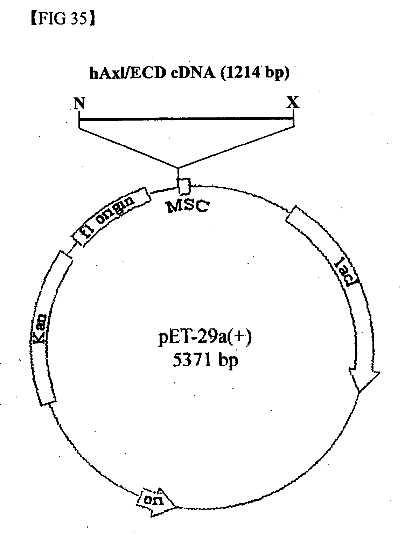

According to the same method as Example 1(1), total RNA was separated from HUVEC(Human Umbilical Vein Endothelial Cell, ATCC) which expresses Axl protein. Using Axl primer (sense; 5'-cca tat gga aag tcc ctt cgt ggg caa c, antisense; 5'-cct cga gca cca gct ggt gga ctg gct g), RT-PCT was carried out to obtain 1214 bp cDNA which corresponds to the extra cellular domain with the signal transfer sequence deleted. It was then cloned into the restriction site between NdeI(1U, Roche) and XhoI(1U) of pET29a (50ng) by using T4 DNA ligase (1U) so that Axl cDNA(100ng) is tagged with (his)6 at its carboxy terminal. Thus obtained clone was named as pET-hAxl/ECD (Fig 35).

### Example 19

### Preparation of human Axl antigen

For the expression of Axl-(his)6 protein, E.Coli BL21(DE3) was transformed with pGEX-4T-3-Axl/F and pGEX-4T-3-Axl/R, respectively. Resulting transformed colonies were inoculated into the media (LB broth, Gibco) comprising kanamycin, an antibiotic. 0.5mM IPTG (Sigma) was further added and the cells were cultured at 25 °C for 4 hrs. Thus obtained floating bacteria cells were centrifuged for 10 min at 6000rpm. Recovered cells were suspended in 1X PBS. After maintained on ice, the cells were completely broken by sonicator and the supernatant was separated by centrifuge at 12,000 rpm for 30min. In order to purify the fusion protein from water soluble and insoluble hAxl/ECD present in the supernatant (i.e., bacteria lysate), the lysate was treated with HiTrapTM chelating HP(Amersham pharmacia). Axl-(his)6 fusion protein was securely obtained.

### Example 20

### Preparation of polyclonal antibody specific to human Axl protein

After obtaining human recombinant Axl antigen from E.Coli, it was used as an antigen to give anti rabbit human Axl antibody. In order to prepare polyclonal antibodies specific to antigen, 300 µg of human Axl protein solubilized in phosphate buffer in concentration of 1mg/ml was emulsified with the same amount of complete Freund's adjuvant, and then the resulting mixture was used for the first immunoinjection to a rabbit. After the first injection, the emulsion comprising the antigen in the same amount as the first injection and incomplete Freund's adjuvant was administered to the animal via muscular injection, for the second, the third and the fourth immunoinjection which were carried out with 2 weeks, 1week and 1 week interval, respectively. Seven days after the fourth injection, sample blood was taken from a hole made into the animal's heart. The blood sample was left at room temperature for 30 min and at 4°C overnight, thus completely coagulated. Supernatant was obtained by the centrifuge at 2500rpm for 30min, which corresponds to blood serum. The resulting serum was diluted five times with 1XPBS and then purified on Protein A column.

### Example 21

### Preparation of monoclonal antibody specific to human Axl protein

Purified human Axl protein was emulsified with the same amount of incomplete Freund's adjuvant, and then the resulting mixture was intraperitoneally injected three times to 6 to 8 week old BALB/c mouse with 2-week interval. After the final injection, formation of the antibody directed against Axl protein was determined by ELISA. After two weeks, the last immunization with 25 µg of human Axl was carried out. Five days later spleen cells were taken from the mouse and mixed with Sp2/0 myeloma cells in 10:1 ratio. Resulting mixture was left inside 50% polyethylene glycol 1500 solution for 3 min to induce cell fusion. After the centrifuge at 1200rpm for 8 min, cellular precipitate obtained was mixed into HAT RPMI-1640 medium comprising 10% fetal bovine serum so that cell concentration became 3.5 x 10⁶ cells per one ml of the medium, and this was seeded to 96-well plate (0.1 ml/well) and incubated in 5% CO₂ incubator at 37°C. After three days of the incubation, 0.1 ml of HAT RPMI-1640 medium comprising 10% fetal bovine serum was added into each well and about half of the medium was replaced with the fresh one every 4 days. ELISA was performed for the culture medium and the cells with high titer were recovered from the plate and cultured with the limiting dilution method. ELISA was performed for the culture of 0.5 cell/well/96-well plate and the hybridoma with antigen specificity and high activity was selected. After culture with HAT selection medium, the ability of hybridoma cells for producing the desired antibody was determined by immunoassay. Specifically, human Axl protein used for the immunization above was diluted to 0.1 µg/ml with 0.01M carbonate-bicarbonate buffer (pH 9.6) and the resulting solution was added to the well 50 µℓ each and coated overnight at 4°C. Then, the wells were washed four times with PBST(phosphate buffer saline, 137mM NaCl, 2.7mM KCl, 10mM Na₂HPO₄, 2mM KH₂PO₄, 0.15 % Tween 20) and blocking was carried out by incubating with 0.1% albumin at 37 °C for 30min. Culture supernatant was added into the well 50 µℓ each and the reaction was carried out for 2 hrs at room temperature. Washing with PBST was carried out four times. Anti-mouse immunoglobulin, which is a secondary antibody tagged with biotin, was diluted with 0.1% BSA-PBST to 1 µg/ml, and added to the well 50 µℓ each and reacted for 1hr at 37°C. Once again, the wells were washed with PBST four times. Streptavidin-Horseradish Peroxidase was diluted 100 times with 0.1% BSA-PBST and introduced to the well 50 µℓ each and reacted for 30min at 37°C. The wells were washed with PBST four times again. As a substrate for enzyme reaction, TMB (Tetra-Methylbenzidine) solution was added to the well 50 µℓ each and reacted at room temperature. After stopping the reaction with 2N sulfuric acid, absorbance was measured at the wavelength of 450nm using ELISA reader. Cells which reacted positively according to the test for anti-human Axl antibody were cultured by subcloning three times (0.3 cells per well) for monoclonalization, thus obtaining hybridoma which produces anti-human Axl monoclonal antibody. To separate anti-human Axl monoclonal antibody from the culture supernatant of the hybridoma, the antibody was purified by GC column and dialyzed. Anti-human Axl monoclonal antibody was finally obtained.

### Example 22

### Construction of human Gas6 expression vector

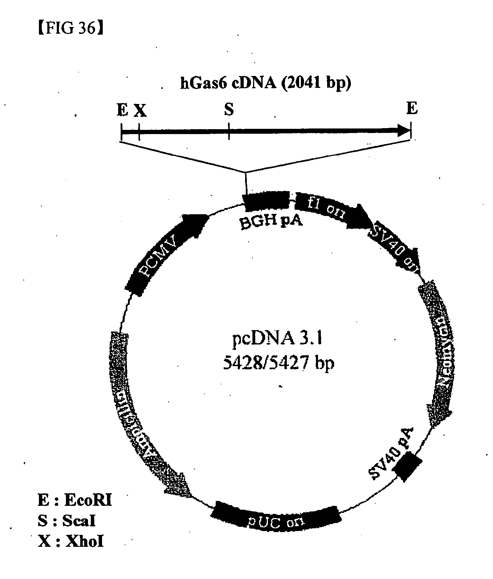

Using the same method as described Example 1(C), total RNA was separated from human cell line HUVEC (American Type Culture collection), which expresses Gas6 protein. Using Gas6 primer (sense; 5'-ggcccgtggccccttcgctct, antisense; ggcctaggctgcggcgggct), RT-PCR was carried out to amplify 2041 bp cDNA, which was then cloned into PCR coning vector. DNA sequencing was carried out for the clone. Analyzed human Gas6 cDNA was digested with EcoRI(1U) and purified. Thus obtained human Gas6 cDNA(100ng) was cloned into pcDNA3.1 vector (50ng), which has been previously digested with EcoRI(1U), using T4 DNA ligase (1U). Consequently pchGas 6 vector which expresses human Gas6 was obtained (Fig 36).

### Example 23

### Preparation of γ-carboxylated human Gas6

To prepare γ-carboxylated human Gas6, human 293 cells (ATCC) were transfected with human Gas6 vector pchGas6 using lipfectamine agent (Invitrogen, Carlslbad, CA). Resulting transfectants were selected in the medium containing 0.75mg/ml of G418 antibiotic (Gibco). By using limiting assay, clones which over-express human Gas6 protein were obtained. 293 cells transfected with human Gas6 secretes a great amount of γ-carboxylated human Gas6 protein into culture supernatant. Western blot analysis of such supernatant according the method described in Example 8 confirms that it corresponds to the clone over-expressing human Gas6 protein. Culture supernatant of 293 cells transfected with human Gas6 was used in 1:20 dilution for inducing the differentiation of human NK cells. Activity of γ-carboxylated human Gas6 was confirmed for this culture supernatant.

### Example 24

### Differentiation of Human Cord Blood-Derived HSCs into mNK cells using Axl polyclonal antibody (Santa Cruz)

### (A) Differentiation of Human Cord Blood-Derived HSCs into mNK cells

HSCs were isolated from cord blood in accordance with the follow method. Human cord blood was divided by 25 mℓ and each contained into 50 mℓ tube. To this was added 25 mℓ of 1 x PBS and gently mixed. Picoll and divided by 20 mℓ and each contained into fresh 50 mℓ tube. The mixture of 1:1 cord blood and 1 x PBS while keeping the layer was poured into the Picoll-containing tube to fill up to 50 mℓ. It was electrophoresed under an unfastened break at 2,000 rpm, 25 °C for 20 minutes. After electrophoresis, white-colored layer (cellular layer = about 10 mℓ) between yellowish top layer (serum) and transparent bottom layer was collected and transferred into 50 mℓ tube containing 20 mℓ of 1 x PBS. It was electrophoresed at 2,000 rpm, 25 °C for 10 minutes. Once the pellet was submerged at the bottom, the supernatant was discarded. The pellet was taken off by tapping with a hand and was mixed with 10 mℓ of ACK buffer solution (0.15 M NH₄Cl, 1 mM KHCO₃, 0.1 mM EDTA (disodium salt), pH 7.2). The mixture was stood at 37 °C for 10 minutes. It was electrophosed at 2,500 rpm, 4°C for 10 minutes. The supernatant was discarded and the pellet was taken off by tapping with a hand. The pellet was suspended into about 2 mℓ of MACS buffer solution (2 mM EDTA and 0.5% BSA were solved in 1 x PBS and filtered) using pipette. The suspension was passed over the filter which had been put on a 50 mℓ tube. The tube containing cells was washed with 10 mℓ of MACS buffer solution and filtered. This working was repeated to make up for 50 mℓ.

Cells contained in 50 mℓ tube were counted using a hemocytometer (Marienfeld). The cells were centrifuged at 2,000 rpm, 4°C for 10 minutes and were counted. The two counts were compared and 1 x 10⁸ was calculated as one reaction. In accordance with the reaction number, the pellet was treated with 1 mℓ of MACS buffer solution. The solution was contained into 1.5 mℓ tube which had been prepared according to the reaction number. It was centrifuged at 1,700 rpm for 3 minutes. After the supernatant was discarded, the pellet was washed by treating a buffer solution. This washing was repeated three times. Finally, the pellet was treated with 500 µℓ MACS solution and was mixed with 25 µℓ of CD34⁺ micro bead and 25 µℓ of blocking reagent in each 1.5 mℓ tube. After reaction at 4 °C for 30 minutes, the tube was turned upside down 10 times every 5 minutes. After 30 minutes, the solution was centrifuged at 1,700 rpm for 3 minutes. The supernatant was washed out three times. Finally, the pellet was treated with 500 µℓ of MACS buffer solution. MACS columns were placed in a MACS and were washed with 3 mℓ of MACS buffer solution. Before the buffer solution was completely flowed, the pellet suspension was carefully poured. Once the pellet suspension was completely flowed, 5 mℓ and 2 mℓ of MACS buffer solutions were successfully flowed.

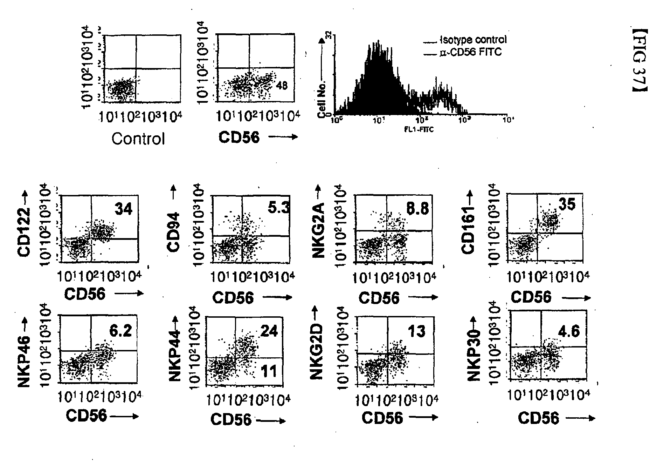

Columns were separated from MACS. 5 mℓ of MACS buffer solution was added and pushed into 15 mℓ tube. 5 mℓ of MACS buffer solution was added once more and pushed into the tube. The cells were counted and plated at a 24-well plate containing 1 mℓ of HSC culture solution (human SCF 30 ng/mℓ (Pepro Tech), human Flt-3L 50 ng/mℓ (Pepro Tech), human IL-7 10 ng/mℓ (Pepro Tech)) at 1 x 10⁶ cells/mℓ per well. The plate was cultured at 37°C, 5% CO₂ incubator. Isolated CD34⁺ HSCs were suspended in Myelocult H5100 (Gibco) medium containing human SCF (30 ng/mℓ), Flt-3L (50 ng/mℓ), and IL-7 (10 ng/mℓ) at 1 x 10⁶/mℓ and were cultured in 24-well plate. The cells were subcultured by replacing half of the medium with a fresh medium every 3 days. These cells were cultured for 14 days to afford human precursor natural killer cells. The resulting pNK cells were co-cultured on MyeloCult medium (Gibco) containing human IL-15 (20 ng/mℓ, Pepro Tech) along with stromal cells isolated from cord blood (isolation method: CD34⁻ HSCs were isolated from cord blood, plated over 10% RPMI medium and cultured in 5% CO₂ incubator for 2 hours. The cells floated on the culture soup were plated on fresh 10% RPMI medium. After 4 and 7 days, culture solutions were replaced. On day 8, after medium was removed and 3 mℓ of 1 x PBS was added, the culture was stood for 5 minutes in incubator. The culture was treated with 1 mℓ of trypsin-EDTA for one minute and the cells were treated with 10% RPMI medium. The cells were collected by centrifugation and stromal cells were plated over 24-well plate at 4 x 10⁴/well.) to afford mature natural killer cells. The purity of the developed mNK cells was analyzed by FACS as described in the above Example 1(B). NKG2A (Pharmingen), CD161 (Pharmingen), NKP46 (Pharmingen), NKP30 (Pharmingen), NKP44 (Pharmingen), NKG2D (Pharmingen), and CD56 (Pharmingen) were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were developed as 8.8%, 35%, 6.2%, 4.6%, 24% and 13%, respectively, as compared to isotype control (Figure 37).

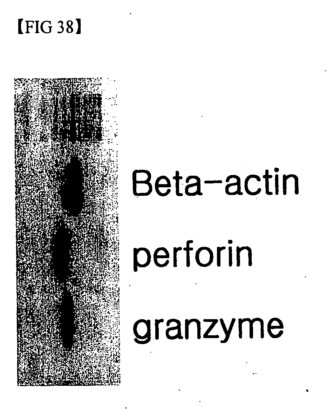

The specific expression of perforin and granzyme genes in the developed mNK cells was ascertained by RT-PCR (Figure 38). The RT-PCR was performed as described in the above Example 1(C).

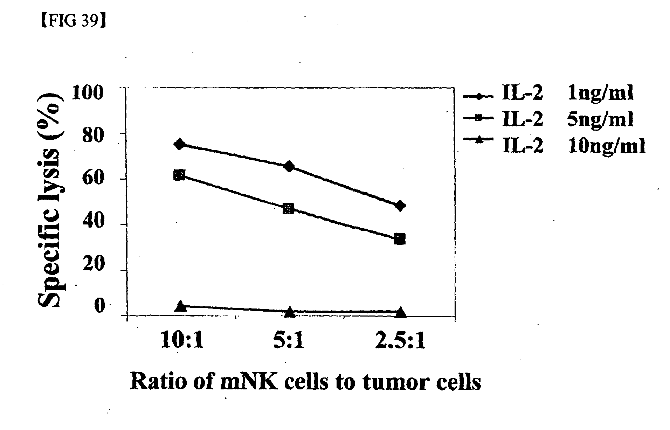

The developed mNK cells were tested for tumor-killing capability. The cells were stimulated with human IL-2 (1, 5 and 10 ng/mℓ) for 62 hours and were reacted with K562 lymphoma cell (ATCC) labeled with ⁵¹Cr (50 uci) for 2 hours at ratio of 10:1, 5:1, and 2.5:1. After 4 hours, the amount of ⁵¹Cr secreted from culture supernatant was measured. As results, tumor-killing capabilities was augmented by increasing the number of mNK cells (Figure 39).

### (B) Differentiation of human pNK cells into mNK cells without stromal cells

In accordance with the same manner as described in the above (A), human pNK cells were differentiated into mNK cells in the absence of human stromal cells. The expression of mNK-associated surface molecules including CD56 (Pharmingen) was analyzed by FACS in accordance with the same method as described in the above Example 1(B). As results, the expression degree in the absence of stromal cells was considerably lower than that under co-culturing with stromal cells. This proves that it is preferred to use stromal cells in the differentiation of pNK cells into mNK cells.

### (C) Differentiation of human cord blood-derived HSCs into mNK cells using Axl polyclonal antibody (Santa Cruz)

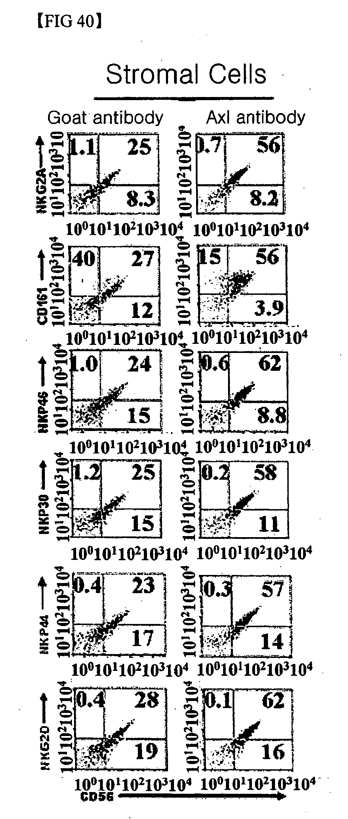

The pNK cells obtained in the above (A) were differentiated in accordance with the same manner as described in the above (A) but using 1 µg/mℓ of Axl polyclonal antibody (Santa Cruz) for 14 days instead of IL-15. The development degree of human mNK cells was ascertained by FACS in accordance with the same method as described in the above Example 1(B). NKG2A (Pharmingen), CD161 (Pharmingen), NKP46 (Pharmingen), NKP30 (Pharmingen), NKP44 (Pharmingen), NKG2D (Pharmingen), and CD56 (Pharmingen) were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using Axl polyclonal antibody than by using goat antibody (control) (Figure 40).

In the same manner as described above, pNK cells were treated with the 1:1 combination (1 µg/mℓ) of Axl polyclonal antibody (Santa Cruz) and IL-15 (Pepro Tech) under the same conditions and the purity was ascertained. As results, the development degree of mNK cells was slightly increased by the combination, in comparison with by Axl polyclonal antibody alone.

### Example 25

### Differentiation of human cord blood-derived HSCs into mNK cells using human Axl polyclonal antibody

The pNK cells obtained in the above Example 24(A) were differentiated in accordance with the same manner as described in the above Example 24(A) but using 1 µg/mℓ of human Axl polyclonal antibody obtained in the above Example 20 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human, mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using human Axl polyclonal antibody than by using goat antibody (control). This result is similar to that differentiated into mNK cells by using commercially available Axl polyclonal antibody.

In the same manner as described above, pNK cells were treated with the 1:1 combination (1 µg/mℓ) of human Axl polyclonal antibody and IL-15 (Pepro Tech) under the same conditions and the purity was ascertained. As results, the development degree of mNK cells was slightly increased by the combination, in comparison with by human Axl polyclonal antibody alone.

### Example 26

### Differentiation of human cord blood-derived HSCs into mNK cells using human Axl monoclonal antibody

The pNK cells obtained in the above Example 24(A) were differentiated in accordance with the same manner as described in the above Example 24(A) but using 1 µg/mℓ of human Axl monoclonal antibody obtained in the above Example 21 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using human Axl monoclonal antibody than by using goat antibody (control). This result is similar to that differentiated into mNK cells by using commercially available Axl polyclonal antibody.

In the same manner as described above, pNK cells were treated with the 1:1 combination (1 µg/mℓ) of human Axl monoclonal antibody and IL-15 (Pepro Tech) under the same conditions and the purity was ascertained. As results, the development degree of mNK cells was slightly increased by the combination, in comparison with by human Axl monoclonal antibody alone.

### Example 27

### Differentiation of human cord blood-derived HSCs into mNK cells using γ-carboxylated human Gas6

The pNK cells obtained in the above Example 24(A) were differentiated in accordance with the same manner as described in the above Example 24(A) but using 1 µg/mℓ of 1:20 diluted culture supernatant of Gas6-transfectant obtained in the above Example 23 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using γ-carboxylated human Gas6 than by using culture supernatant of vector-transfected cell (control). This result is similar to that differentiated into mNK cells by using human Axl polyclonal antibody or Axl monoclonal antibody.

In the same manner as described above, pNK cells were treated with the 1:1 combination (1 µg/mℓ) of γ-carboxylated human Gas6 and IL-15 (Pepro Tech) under the same conditions and the purity was ascertained. As results, the development degree of mNK cells was slightly increased by the combination, in comparison with by γ-carboxylated human Gas6 alone.

### Example 28

### Differentiation of human cord blood-derived HSCs into mNK cells using Axl polyclonal antibody (Santa Cruz) and γ-carboxylated human Gas6

The pNK cells obtained in the above Example 24(A) were differentiated in accordance with the same manner as described in the above Example 24(A) but using 1 µg/mℓ of Axl polyclonal antibody (Santa Cruz) and 1:20 diluted culture supernatant of Gas6-transfectant obtained in the above Example 23 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were more slightly developed by using the combination of γ-carboxylated human Gas6 and Axl polyclonal antibody than by using γ-carboxylated human Gas6 alone or Axl polyclonal antibody alone (control).

In the same manner as described above, pNK cells were treated with the combination of Axl polyclonal antibody and γ-carboxylated human Gas6 along with IL-15(1:1 ratio with antibody) under the same conditions and the purity was ascertained. As results, the development degree of mNK cells was slightly increased by the combination of the three ingredients, in comparison with by the combination of Axl polyclonal antibody and γ-carboxylated human Gas6.

### Example 29

### Differentiation of human cord blood-derived HSCs into mNK cells using human Axl polyclonal antibody and γ-carboxylated human Gas6

The pNK cells obtained in the above Example 24(A) were differentiated in accordance with the same manner as described in the above Example 24(A) but using human Axl polyclonal antibody obtained in the above Example 20 and γ-carboxylated human Gas6 obtained in the above Example 23 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were more slightly developed by using the combination of γ-carboxylated human Gas6 and human Axl polyclonal antibody than by using γ-carboxylated human Gas6 alone or human Axl polyclonal antibody alone (control). This result is similar to that differentiated into mNK cells by using the combination of commercially available Axl polyclonal antibody and γ-carboxylated human Gas6.

In the same manner as described above, pNK cells were treated with the combination of Axl polyclonal antibody and γ-carboxylated human Gas6 along with IL-15 (1:1 ratio with antibody) under the same conditions and the purity was ascertained. As results, the development degree of mNK cells was slightly increased by the combination of the three ingredients, in comparison with by the combination of human Axl polyclonal antibody and γ-carboxylated human Gas6.

### Example 30

### Differentiation of human cord blood-derived HSCs into mNK cells using human Axl monoclonal antibody and γ-carboxylated human Gas6

The pNK cells obtained in the above Example 24(A) were differentiated in accordance with the same manner as described in the above Example 24(A) but using human Axl monoclonal antibody obtained in the above Example 21 and γ-carboxylated human Gas6 obtained in the above Example 23 for 14 days instead of IL-15. The development degree of human mNK cells was ascertained by FACS in accordance with the same method as described in the above Example 1(B). NKG2A, CD 161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were more slightly developed by using the combination of γ-carboxylated human Gas6 and human Axl monoclonal antibody than by using γ-carboxylated human Gas6 alone or human Axl monoclonal antibody alone (control). This result is similar to that differentiated into mNK cells by using the combination of commercially available Axl polyclonal antibody and γ-carboxylated Gas6.

In the same manner as described above, pNK cells were treated with the combination of Axl monoclonal antibody and γ-carboxylated human Gas6 along with IL-15 (1:1 ratio with antibody) under the same conditions and the purity was ascertained. As results, the development degree of mNK cells was slightly increased by the combination of the three ingredients, in comparison with by the combination of human Axl monoclonal antibody and γ-carboxylated human Gas6.

### Example 31

### Anti-tumor activity in an animal tumor model of mNK cells developed from human cord blood-derived HSCs by Axl polyclonal antibody (Santa Cruz)

5-week-old male nude Balb/c mice (immunodeficient transgenic mice purchased from Orient Bio) were used to see whether human mNK cells developed by Axl polyclonal antibody (Santa Cruz) exert tumor-killing activity. The nude mice were housed in a germ-free animal room according to an animal management guideline. Human tumor cell lines (for example, gastric carcinoma KCLB cat. no. 00638, uterine carcinoma KCLB cat. no. 10002, breast carcinoma KCLB cat. no. 30022, renal carcinoma KCLB cat. no. 30044, melanoma KCLB cat. no. 30068, lung carcinoma KCLB cat. no. 30053, ovarian carcinoma KCLB cat. no. 30077, etc.) were cultured on RPMI 1640 media (Gibco) containing 10% fetal bovine serum at 37 °C in CO₂ incubator. The cultured tumor cell lines were washed with 1 x PBS and were treated with trypsin-EDTA (Gibco). The trpsin-EDTA was removed from cell lines which was then suspended in 1 x PBS. The cells were counted to prepare 10⁸ cells/0.1 µℓ. Nude mice were subcutaneously injected with 10⁸ tumor cells by an insulin syringe (Pharmingen) and were grown for about one week until they were about 0.8 mm in diameter. Human mNK cells (1 x 10⁶) developed by Axl polyclonal antibody (Santa Cruz) were activated with human IL-2 (10 ng/mℓ). The formed tumor tissue site was injected with the activated mNK cells. Tumor volume was measured every hour. As results, tumor volume of the test group injected with human mNK cells developed by Axl polyclonal antibody (Santa Cruz) was remarkably reduced in comparison with that of the control group injected with human mNK cells developed by goat antibody.

### Example 32

### Anti-tumor activity in an animal tumor model of mNK cells developed from human cord blood-derived HSCs by human Axl polyclonal antibody

The same experimental method as described in the above Example 31 was conducted to see whether human mNK cells developed by human Axl polyclonal antibody exert tumor-killing activity. As results, tumor volume of the test group injected with human mNK cells developed by human Axl polyclonal antibody was remarkably reduced in comparison with that of the control group injected with human mNK cells developed by goat antibody. This is similar with the result obtained by using mNK cells developed by commercially available Axl polyclonal antibody.

### Example 33

### Anti-tumor activity in an animal tumor model of mNK cells developed from human cord blood-derived HSCs by human Axl monoclonal antibody

The same experimental method as described in the above Example 31 was conducted to see whether human mNK cells developed by human Axl monoclonal antibody exert tumor-killing activity. As results, tumor volume of the test group injected with human mNK cells developed by human Axl monoclonal antibody was remarkably reduced in comparison with that of the control group injected with human mNK cells developed by goat antibody. This is similar with the result obtained by using mNK cells developed by commercially available Axl polyclonal antibody.

### Example 34

### Anti-tumor activity in an animal tumor model of mNK cells developed from human cord blood-derived HSCs by γ-carboxylated human Gas6

The same experimental method as described in the above Example 31 was conducted to see whether human mNK cells developed by γ-carboxylated human Gas6 exert tumor-killing activity. As results, tumor volume of the test group injected with human mNK cells developed by γ-carboxylated human Gas6 was remarkably reduced in comparison with that of the control group injected with human mNK cells developed by culture supernatant of vector-containing transfectant. This is similar with the result obtained by using mNK cells developed by commercially available Axl polyclonal antibody.

### Example 35

### Anti-tumor activity in an animal tumor model of mNK cells developed from human cord blood-derived HSCs by the combination of Axl polyclonal antibody (Santa Cruz) and γ-carboxylated human Gas6

The same experimental method as described in the above Example 31 was conducted to see whether human mNK cells developed by the combination of Axl polyclonal antibody (Santa Cruz) and γ-carboxylated human Gas6 exert tumor-killing activity. As results, tumor volume of the test group injected with human mNK cells developed by the combination of Axl polyclonal antibody (Santa Cruz) and γ-carboxylated human Gas6 was more slightly reduced in comparison with that of the control group injected with human mNK cells developed by Axl polyclonal antibody (Santa Cruz) or the control group injected with human mNK cells developed by culture supernatant of vector-containing transfectant.

### Example 36

### Anti-tumor activity in an animal tumor model of mNK cells developed from human cord blood-derived HSCs by the combination of human Axl polyclonal antibody and γ-carboxylated human Gas6

The same experimental method as described in the above Example 31 was conducted to see whether human mNK cells developed by the combination of human Axl polyclonal antibody and γ-carboxylated human Gas6 exert tumor-killing activity. As results, tumor volume of the test group injected with human mNK cells developed by the combination of human Axl polyclonal antibody and γ-carboxylated human Gas6 was more slightly reduced in comparison with that of the control group injected with human mNK cells developed by human Axl polyclonal antibody or the control group injected with human mNK cells developed by culture supernatant of vector-containing transfectant. This is similar with the result obtained by using mNK cells developed by the combination of commercially available Axl polyclonal antibody and culture supernatant of γ-carboxylated human Gas6-containing transfectant.

### Example 37

### Anti-tumor activity in an animal tumor model of mNK cells developed from human cord blood-derived HSCs by the combination of human Axl monoclonal antibody and γ-carboxylated human Gas6

The same experimental method as described in the above Example 31 was conducted to see whether human mNK cells developed by the combination of human Axl monoclonal antibody and γ-carboxylated human Gas6 exert tumor-killing activity. As results, tumor volume of the test group injected with human mNK cells developed by the combination of human Axl monoclonal antibody and γ-carboxylated human Gas6 was more slightly reduced in comparison with that of the control group injected with human mNK cells developed by human Axl monoclonal antibody or the control group injected with human mNK cells developed by culture supernatant of vector-containing transfectant. This is similar with the result obtained by using mNK cells developed by the combination of commercially available Axl polyclonal antibody and culture supernatant of γ-carboxylated human Gas6-containing transfectant.

### Example 38

### Differentiation of Human Bone Marrow-Derived HSCs into mNK cells using Axl polyclonal antibody (Santa Cruz)

### (A) Differentiation of Human Bone Marrow-Derived HSCs into mNK cells

Human bone marrow was divided by 25 mℓ which was then contained into 50 mℓ tube. In accordance with the same method as described in the above Example 24(A), HSCs were differentiated via pNK cells into mNK cells. Human stromal cells used in the procedure were isolated from human bone marrow in accordance with the same method as described in the above Example 24(A) for the isolation of stromal cells from cord blood.

### (B) Development of mNK cells using Axl polyclonal antibody (Santa Cruz)

The pNK cells obtained in the above (A) were differentiated in accordance with the same manner as described in the above (A) but using 1 µg/mℓ of Axl polyclonal antibody (Santa Cruz) for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using Axl polyclonal antibody than by using goat antibody (control).

In the same manner as described above, pNK cells were treated with the 1:1 combination (1 µg/mℓ) of Axl polyclonal antibody (Santa Cruz) and IL-15 (Pepro Tech) under the same conditions and the purity was ascertained. As results, the development degree of mNK cells was slightly increased by the combination, in comparison with by Axl polyclonal antibody alone.

### Example 39

### Differentiation of human bone marrow-derived HSCs into mNK cells using human Axl polyclonal antibody

The pNK cells obtained in the above Example 38(A) were differentiated in accordance with the same manner as described in the above Example 38(A) but using 1 µg/mℓ of human Axl polyclonal antibody for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results; CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using human Axl polyclonal antibody than by using goat antibody (control). This result is similar to that differentiated into mNK cells by using commercially available Axl polyclonal antibody.

In the same manner as described above, pNK cells were treated with the 1:1 combination (1 µg/mℓ) of human Axl polyclonal antibody and IL-15 (Pepro Tech) under the same conditions and the purity was ascertained. As results, the development degree of mNK cells was slightly increased by the combination, in comparison with by Axl polyclonal antibody alone.

### Example 40

### Differentiation of human bone marrow-derived HSCs into mNK cells using human Axl monoclonal antibody

The pNK cells obtained in the above Example 38(A) were differentiated in accordance with the same manner as described in the above Example 38(A) but using 1 µg/mℓ of human Axl monoclonal antibody for 14 days instead of IL-15. The development degree of human mNK cells was ascertained by FACS in accordance with the same method as described in the above Example 1(B). NKG2A, CD161, NK46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using human Axl monoclonal antibody than by using goat antibody (control). This result is similar to that differentiated into mNK cells by using commercially available Axl polyclonal antibody.

In the same manner as described above, pNK cells were treated with the 1:1 combination (1 µg/mℓ) of human Axl monoclonal antibody and IL-15 (Pepro Tech) under the same conditions and the purity was ascertained. As results, the development degree of mNK cells was slightly increased by the combination, in comparison with by Axl monoclonal antibody alone.

### Example 41

### Differentiation of human bone marrow-derived HSCs into mNK cells using γ-carboxylated human Gas6

The pNK cells obtained in the above Example 38(A) were differentiated in accordance with the same manner as described in the above Example 38(A) but using 1:20 diluted culture supernatant of Gas6-containing transfectant produced in the above Example 23 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were about two to three times more developed by using γ-carboxylated human Gas6 than by using culture supernatant of vector-containing transfectant (control). This result is similar to that differentiated into mNK cells by using human Axl polyclonal antibody or Axl monoclonal antibody.

In the same manner as described above, pNK cells were treated with the 1:1 combination (1 µg/mℓ) of γ-carboxylated human Axl and IL-15 (Pepro Tech) under the same conditions and the purity was ascertained. As results, the development degree of mNK cells was slightly increased by the combination, in comparison with by γ-carboxylated human Gas6 alone.

### Example 42

### Differentiation of human bone marrow-derived HSCs into mNK cells using the combination of Axl polyclonal antibody (Santa Cruz) and γ-carboxylated human Gas6

The pNK cells obtained in the above Example 38(A) were differentiated in accordance with the same manner as described in the above Example 38(A) but using the combination of Axl polyclonal antibody (Santa Cruz) and 1:20 diluted culture supernatant of Gas6-containing transfectant produced in the above Example 23 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were slightly more developed by using the combination of Axl polyclonal antibody (Santa Cruz) and 1:20 diluted culture supernatant of Gas6-containing transfectant than by using Axl polyclonal antibody (Santa Cruz) alone (control) or 1:20 diluted culture supernatant of Gas6-containing transfectant alone (control).

In the same manner as described above, pNK cells were treated with the combination of Axl polyclonal antibody (Santa Cruz) and 1:20 diluted culture supernatant of Gas6-containing transfectant along with IL-15 (Pepro Tech) (1:1 ratio of antibody) under the same conditions and the purity was ascertained. As results, the development degree of mNK cells was slightly increased by the combination of the above three ingredients, in comparison with by the combination of Axl polyclonal antibody and γ-carboxylated human Gas6.

### Example 43

### Differentiation of human bone marrow-derived HSCs into mNK cells using the combination of human Axl polyclonal antibody and γ-carboxylated human Gas6

The pNK cells obtained in the above Example 38(A) were differentiated in accordance with the same manner as described in the above Example 38(A) but using the combination of 1 µg/mℓ of human Axl polyclonal antibody produced in the above Example 19 and 1:20 diluted culture supernatant of Gas6-containing transfectant produced in the above Example 23 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were slightly more developed by using the combination of human Axl polyclonal antibody and 1:20 diluted culture supernatant of Gas6-containing transfectant than by using human Axl polyclonal antibody alone (control) or 1:20 diluted culture supernatant of Gas6-containing transfectant alone (control). This result is similar to that differentiated into mNK cells by using the combination of commercially available Axl polyclonal antibody and culture supernatant of Gas6-containing transfectant.

In the same manner as described above, pNK cells were treated with the combination of human Axl polyclonal antibody and γ-carboxylated human Gas6 along with IL-15 (Pepro Tech) (1:1 ratio of the antibody) under the same conditions and the purity was ascertained. As results, the development degree of mNK cells was slightly increased by the combination of the above three ingredients, in comparison with by the combination of human Axl polyclonal antibody and γ-carboxylated human Gas6.

### Example 44

### Differentiation of human bone marrow-derived HSCs into mNK cells using the combination of human Axl monoclonal antibody and γ-carboxylated human Gas6

The pNK cells obtained in the above Example 38(A) were differentiated in accordance with the same manner as described in the above Example 38(A) but using the combination of 1 µg/mℓ of human Axl monoclonal antibody produced in the above Example 21 and 1:20 diluted culture supernatant of Gas6-containing transfectant produced in the above Example 23 for 14 days instead of IL-15. The development degree of human mNK cells was ascertained by FACS in accordance with the same method as described in the above Example 1(B). NKG2A, CD161, NKP46, NKP30, NKP44, NKG2D, and CD56 were used as markers for human mNK cells. As results, CD56⁺NKG2A⁺, CD56⁺CD161⁺, CD56⁺NKP46⁺, CD56⁺NKP30⁺, CD56⁺NKP44⁺ and CD56⁺NKG2D⁺ cells were slightly more developed by using the combination of human Axl monoclonal antibody and culture supernatant of Gas6-containing transfectant than by using human Axl monoclonal antibody alone (control) or culture supernatant of Gas6-containing transfectant alone (control). This result is similar to that differentiated into mNK cells by using the combination of commercially available Axl polyclonal antibody and culture supernatant of Gas6-containing transfectant.

In the same manner as described above, pNK cells were treated with the combination of Axl monoclonal antibody and γ-carboxylated human Gas6 along with IL-15 (Pepro Tech) (1:1 ratio of the antibody) under the same conditions and the purity was ascertained. As results, the development degree of mNK cells was slightly increased by the combination of the above three ingredients, in comparison with by the combination of human Axl monoclonal antibody and γ-carboxylated human Gas6.

### Example 45

### Anti-tumor activity in an animal tumor model of mNK cells developed from human bone marrow-derived HSCs by Axl polyclonal antibody (Santa Cruz)

The same experimental method as described in the above Example 31 was conducted to see whether human mNK cells developed by Axl polyclonal antibody (Santa Cruz) exert tumor-killing activity. As results, tumor volume of the test group injected with human mNK cells developed by Axl polyclonal antibody (Santa Cruz) was remarkably reduced in comparison with that of the control group injected with human mNK cells developed by goat antibody.

### Example 46

### Anti-tumor activity in an animal tumor model of mNK cells developed from human bone marrow-derived HSCs by human Axl polyclonal antibody

The same experimental method as described in the above Example 31 was conducted to see whether human mNK cells developed by human Axl polyclonal antibody exert tumor-killing activity. As results, tumor volume of the test group injected with human mNK cells developed by human Axl polyclonal antibody was remarkably reduced in comparison with that of the control group injected with human mNK cells developed by goat antibody. This is similar with the result obtained by using mNK cells developed by commercially available Axl polyclonal antibody.

### Example 47

### Anti-tumor activity in an animal tumor model of mNK cells developed from human bone marrow-derived HSCs by human Axl monoclonal antibody

The same experimental method as described in the above Example 31 was conducted to see whether human mNK cells developed by human Axl monoclonal antibody exert tumor-killing activity. As results, tumor volume of the test group injected with human mNK cells developed by human Axl monoclonal antibody was remarkably reduced in comparison with that of the control group injected with human mNK cells developed by goat antibody. This is similar with the result obtained by using mNK cells developed by commercially available Axl polyclonal antibody.

### Example 48

### Anti-tumor activity in an animal tumor model of mNK cells developed from human bone marrow-derived HSCs by γ-carboxylated human Gas6

The same experimental method as described in the above Example 31 was conducted to see whether human mNK cells developed by γ-carboxylated human Gas6 exert tumor-killing activity. As results, tumor volume of the test group injected with human mNK cells developed by γ-carboxylated human Gas6 was remarkably reduced in comparison with that of the control group injected with human mNK cells developed by culture supernatant of vector-containing transfectant. This is similar with the result obtained by using mNK cells developed by commercially available Axl polyclonal antibody.

### Example 49

### Anti-tumor activity in an animal tumor model of mNK cells developed from human bone marrow-derived HSCs by the combination of Axl polyclonal antibody (Santa Cruz) and γ-carboxylated human Gas6

The same experimental method as described in the above Example 31 was conducted to see whether human mNK cells developed by the combination of Axl polyclonal antibody (Santa Cruz) and γ-carboxylated human Gas6 exert tumor-killing activity. As results, tumor volume of the test group injected with human mNK cells developed by the combination of Axl polyclonal antibody (Santa Cruz) and γ-carboxylated human Gas6 was more slightly reduced in comparison with that of the control group injected with human mNK cells developed by Axl polyclonal antibody (Santa Cruz) or the control group injected with human mNK cells developed by culture supernatant of vector-containing transfectant.

### Example 50

### Anti-tumor activity in an animal tumor model of mNK cells developed from human bone marrow-derived HSCs by the combination of human Axl polyclonal antibody and γ-carboxylated human Gas6

The same experimental method as described in the above Example 31 was conducted to see whether human mNK cells developed by the combination of human Axl polyclonal antibody and γ-carboxylated human Gas6 exert tumor-killing activity. As results, tumor volume of the test group injected with human mNK cells developed by the combination of human Axl polyclonal antibody and γ-carboxylated human Gas6 was more slightly reduced in comparison with that of the control group injected with human mNK cells developed by human Axl polyclonal antibody or the control group injected with human mNK cells developed by culture supernatant of vector-containing transfectant. This is similar with the result obtained by using mNK cells developed by the combination of commercially available Axl polyclonal antibody and culture supernatant of γ-carboxylated human Gas6-containing transfectant.

### Example 51

### Anti-tumor activity in an animal tumor model of mNK cells developed from human bone marrow-derived HSCs by the combination of human Axl monoclonal antibody and γ-carboxylated human Gas6

The same experimental method as described in the above Example 31 was conducted to see whether human mNK cells developed by the combination of human Axl monoclonal antibody and γ-carboxylated human Gas6 exert tumor-killing activity. As results, tumor volume of the test group injected with human mNK cells developed by the combination of human Axl monoclonal antibody and γ-carboxylated human Gas6 was more slightly reduced in comparison with that of the control group injected with human mNK cells developed by human Axl monoclonal antibody or the control group injected with human mNK cells developed by culture supernatant of vector-containing transfectant. This is similar with the result obtained by using mNK cells developed by the combination of commercially available Axl polyclonal antibody and culture supernatant of γ-carboxylated human Gas6-containing transfectant.

### Example 52

### Differentiation of human peripheral blood-derived HSCs into mNK cells using Axl polyclonal antibody (Santa Cruz)

### (A) Differentiation of human peripheral blood-derived HSCs into mNK cells

Human peripheral blood was divided by 25 mℓ which was then contained into 50 mℓ tube. In accordance with the same method as described in the above Example 24(A), HSCs were differentiated via pNK cells into mNK cells. Human stromal cells used in the procedure were isolated from human peripheral blood in accordance with the same method as described in the above Example 24(A) for the isolation of stromal cells from cord blood.

### (B) Development of mNK cells using Axl polyclonal antibody (Santa Cruz)

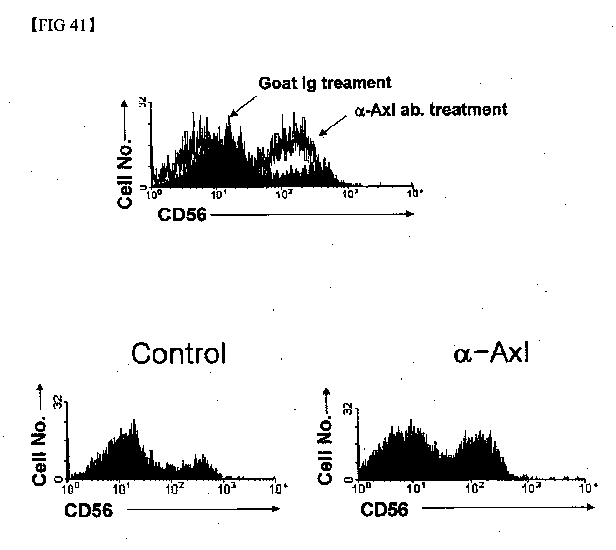

The pNK cells obtained in the above (A) were differentiated in accordance with the same manner as described in the above (A) but using 1 µg/mℓ of Axl polyclonal antibody (Santa Cruz) for 14 days instead of IL-15. The development degree of human mNK cells was ascertained by FACS in accordance with the same method as described in the above Example 1(B). CD56 were used as markers for human mNK cells. As results, CD56⁺ cells were about two to three times more developed by using Axl polyclonal antibody than by using goat antibody (control) (Figure 41).

In the same manner as described above, pNK cells were treated with the 1:1 combination (1 µg/mℓ) of Axl polyclonal antibody (Santa Cruz) and IL-15 (Pepro Tech) under the same conditions and the purity was ascertained. As results, the development degree of mNK cells was slightly increased by the combination, in comparison with by Axl polyclonal antibody alone.

### Industrial Applicability

### Clinical Trial

Two colon cancer patients with distant metastasis, one breast cancer patient recurred with lung metastasis, one non-Hodgkin's lymphoma patient, and one acute lymphocytic leukemia patient were volunteered to take part in this immunotherapy. The patients received G-CSF starting 1 to 5 days after completion of chemotherapy according to the individual treatment protocol in dosages of 600-900 µg/d s.c. until the end of the collection period. CD34⁺ PBSCs were monitored daily as soon as the WBC recovered (>1 x 10⁹/1 PB). Blood sample was taken from the patients when there were 20 x 10⁶ CD34⁺ cells/1 PB. The absolute number of CD34⁺ cells was evaluated by flow cytometry using a FACScan analyzer (Becton Dickinson / Aria) and appropriate isotype-matched, negative control. As described in the above Example 23, the hematopoietic cells were separated from human peripheral blood and differentiated into mature natural killer cells, which were then activated with 10 ng/ml of IL-2. The resulting mNK cells were injected to the patient for autoimmune therapy. Although evidencing examples are not many, substantial decrease in tumor size was observed for all patients.

## Claims

1. A process for producing mature natural killer cell *in vitro* comprising (i) treating hematopoietic stem cell with interleukin-7, stem cell factor and Flt3L to differentiate into precursor natural killer cell, and (ii) treating the resulting precursor natural killer cell with ligand of Axl protein selected from a group consisting of an antibody directed against Axl protein, a γ-carboxylated Gas6 protein and a combination of both to produce mature natural killer cell, and therefore obtaining mature natural killer cell.

2. The process for producing mature natural killer cell according to claim 1 wherein said hematopoietic stem cell is derived from one selected from a group consisting of human bone marrow, peripheral blood, and umbilical cord blood.

3. The process for producing mature natural killer cell according to claim 1 wherein said precursor natural killer cell is treated with the ligand in the presence of human stromal cell.

4. A process for producing activated mature natural killer cell *in vitro* comprising (i) treating hematopoietic stem cell with interleukin-7, stem cell factor and Flt3L to differentiate into precursor natural killer cell, (ii) treating the resulting precursor natural killer cell with Axl antibody to produce mature natural killer cell, and (iii) activating the resulting mature natural killer cell with the treatment of interleukin-2.

5. The process for producing activated mature natural killer cell according to claim 4 wherein interleukin-2 is used in an amount of from 8 to 15 ng/ml.

## Patentansprüche

1. Verfahren zum Herstellen einer reifen natürlichen Killerzelle *in vitro*, umfassend
(i) Behandeln blutbildender Stammzelle mit Interleukin-7, Stammzellenfaktor und Flt3L, um zu einer natürlichen Killervorläuferzelle zu differenzieren und
(ii) Behandeln der resultierenden natürlichen Killervorläuferzelle mit dem Ligand des Axl Proteins, welches aus einer aus einem gegen das Axl-Protein gerichteten Antikörper, einem γ-carboxyliertes Gas6-Protein und einer Kombination von beiden bestehenden Gruppe ausgewählt wird, zur Herstellung einer reifen natürlichen Killerzelle und demzufolge zur Gewinnung einer reifen natürlichen Killerzelle.

2. Verfahren zur Herstellung einer reifen natürlichen Killerzelle nach Anspruch 1, wobei die blutbildende Stammzelle abgeleitet ist aus einem aus der aus menschlichem Knochenmark, peripherem Blut und Nabelschnurblut bestehenden Gruppe.

3. Verfahren zur Herstellung einer reifen natürlichen Killerzelle nach Anspruch 1, wobei die natürliche Killervorläuferzelle mit dem Ligand in Anwesenheit einer menschlichen Bindegewebszelle behandelt wird.

4. Verfahren zur Herstellung einer aktivierten reifen natürlichen Killerzelle *in vitro,* umfassend:
(i) Behandeln blutbildender Stammzelle mit Interleukin-7, Stammzellenfaktor und Flt3L, um zu einer natürlichen Killervorläuferzelle zu differenzieren,
(ii) Behandeln der resultierenden Killervorläuferzelle mit Axl-Antikörper zur Herstellung einer reifen natürlichen Killerzelle und
(iii) Aktivieren der resultierenden reifen natürlichen Killerzelle durch Behandeln mit Interleukin-2.

5. Verfahren zur Herstellung einer aktivierten reifen natürlichen Killerzelle nach Anspruch 4, wobei Interleukin-2 in einer Menge von 8 bis 15 ng/ml benutzt wird.

## Revendications

1. Procédé de production *in vitro* d'une cellule tueuse naturelle mature comprenant (i) le traitement d'une cellule souche hématopoïétique par de l'interleukine-7, du facteur de cellule souche et Flt3L pour la différencier en une cellule tueuse naturelle précurseur et (ii) le traitement de la cellule tueuse naturelle précurseur obtenue par un ligand de la protéine Axl sélectionné dans un groupe constitué d'un anticorps dirigé contre la protéine Axl, une protéine Gas6 γ-carboxylée et leur combinaison pour produire une cellule tueuse naturelle mature, et par conséquent obtenir une cellule tueuse naturelle mature.

2. Procédé de production d'une cellule tueuse naturelle mature selon la revendication 1, où ladite cellule souche hématopoïétique provient d'une cellule sélectionnée dans un groupe constitué de la moelle osseuse humaine, du sang périphérique et du sang de cordon ombilical.

3. Procédé de production d'une cellule tueuse naturelle mature selon la revendication 1, où ladite cellule tueuse naturelle précurseur est traitée par le ligand en présence d'une cellule stromale humaine.

4. Procédé de production *in vitro* d'une cellule tueuse naturelle mature activée comprenant (i) le traitement d'une cellule souche hématopoïétique par de l'interleukine-7, du facteur de cellule souche et FIt3L pour la différencier en une cellule tueuse naturelle précurseur, (ii) le traitement de la cellule tueuse naturelle précurseur obtenue par un anticorps Axl pour produire une cellule tueuse naturelle mature et (iii) l'activation de la cellule tueuse naturelle mature obtenue par traitement par de l'interleukine-2.

5. Procédé de production d'une cellule tueuse naturelle mature activée selon la revendication 4, où l'interleukine-2 est utilisée en une quantité de 8 à 15 ng/ml.
